# EUROPÄISCHE PATENTSCHRIFT

(11) **EP 3 821 247 B1**
(45) Veröffentlichungstag und Bekanntmachung des Hinweises auf die Patenterteilung: **28.12.2022**
(21) Anmeldenummer: 19742670.3
(22) Anmeldetag: 11.07.2019
(51) Int. Cl.: G01N 33/53, G01N 33/543

(54) **VERFAHREN UND VORRICHTUNG ZUR DURCHFÜHRUNG VON HETEROGENEN IMMUNOASSAYS**
METHOD AND APPARATUS FOR PERFORMING HETEROGENEOUS IMMUNOASSAY
PROCÉDÉ ET DISPOSITIF POUR L'EXÉCUTION DE DOSAGES IMMUNOLOGIQUES HÉTÉROGÈNES

(30) Priorität: 13.07.2018 AT 506032018
(43) Veröffentlichungstag der Anmeldung: 19.05.2021
(73) Patentinhaber: MEON Medical Solutions GmbH & Co KG, 8010 Graz (AT)
(72) Erfinder: SPRENGERS, Wolfgang, 8081 VASOLDSBERG (AT); BARTEL, Arnold, 8051 GRAZ (AT); MARIK, Reinhard, 8020 GRAZ (AT)
(74) Vertreter: Babeluk, Michael
(86) Internationale Anmeldenummer: PCT/AT2019/060230
(87) Internationale Veröffentlichungsnummer: WO 2020/010379

(56) Entgegenhaltungen:
- EP-A1- 0 644 426
- EP-A1- 2 502 082
- EP-A2- 0 733 905
- WO-A1-2019/010514

## Beschreibung

Die Erfindung betrifft ein Verfahren und eine Vorrichtung zur Durchführung von heterogenen Immunoassays mit Hilfe magnetischer Partikel in aneinandergereihten Küvetten eines Analysators, wobei jede Küvette eine Füllöffnung und zumindest ein seitliches, für die Messstrahlung durchlässiges Messfenster aufweist.

Heterogene Immunoassays werden routinemäßig, beispielsweise in der klinischen Diagnostik, der Analytik und der Mikrobiologie, verwendet, wobei die Notwendigkeit besteht, verschiedene Eigenschaften und Inhaltsstoffe von flüssigen Proben vor allem mit optischen Verfahren schnell, exakt und reproduzierbar zu bestimmen.

Bei den bekannten Vorrichtungen zur Durchführung von heterogenen Immunoassays kommen unterschiedliche Messprinzipien zur Anwendung. In rotatorisch organisierten Systemen werden Geräte mit einer stationären Detektionseinheit, beispielsweise einem stationären Fotomultiplier, sowie einer Fördereinrichtung zur Bewegung von Küvetten zur Aufnahme der zu vermessenden Reaktionsgemische aus Proben und Reagenzien, wie beispielsweise einem Förderband oder einem Karussell verwendet. Die Küvetten werden sukzessive in starrem Zyklus und starrer Reihenfolge an der Detektionseinheit vorbeigeführt und durchgemessen. Demzufolge muss die Fördereinrichtung jedes Mal anhalten, wenn eine neue Probe oder ein Reagenz in eine Küvette eingebracht wird oder die Küvette getauscht oder gewaschen und für einen neuen Test bereitgestellt werden soll. Mit den konzeptionell starr vorgegebenen Zykluszeiten geht eine deutliche Effizienzeinbuße einher.

Zum besseren Verständnis der Erfindung werden einige wesentliche, in der gegenständlichen Anmeldung verwendete, technische Begriffe näher definiert:
Analyt/Analytmolekül/Antigen: Als Analyt - im Fall von Immunoassays auch als Antigen bezeichnet - wird hier ein, in einer Probe qualitativ und/oder quantitativ zu bestimmender Inhaltsstoff bezeichnet. Der Analyt liegt bei Immunoassays in einer flüssigen Phase vor, meist gelöst in einem Puffer, in verdünnten Körperflüssigkeiten oder anderen Probenflüssigkeiten. Darüber hinaus kann der Analyt auch eine, in einer Suspension vorliegende, durch Immunoassays nachweisbare partikuläre Struktur mit antigenen Oberflächenmerkmalen, wie zum Beispiel Bakterien, Viren, Zellen oder Materialpartikel sein.

Lumineszenz/Chemolumineszenz: Bei analytischen Bestimmungsverfahren mittels Lumineszenz (z.B. Fluoreszenz, Phosphoreszenz, Chemolumineszenz (Englisch: chemiluminescence)) wird das von Molekülen emittierte Licht gemessen. Im Fall der Chemolumineszenz erfolgt die Lichtemission infolge einer chemischen Reaktion. Luminometrische Methoden sind sehr empfindlich und daher gut für die Detektion von Markern in Immunoassays geeignet.

Immunoassay: Als Immunoassay (deutsch auch.: *Immunassay*) werden zusammenfassend eine Reihe von Methoden in der Bioanalytik bezeichnet, deren gemeinsames Grundprinzip die Erkennung und damit der Nachweis eines Analyten (Antigen) in einer flüssigen Phase durch die Bindung des Analyten an einen Antikörper ist. Immunoassays werden beispielsweise in der Labormedizin für die Bestimmung einer Vielzahl von Analyten in verschiedenen Körperflüssigkeiten wie Blut, Serum, Urin oder Liquor verwendet. Dabei dienen sie der Vorhersage, der Diagnose und der Verlaufskontrolle von Krankheiten ebenso wie dem Nachweis von Giftstoffen oder der Überwachung von Arzneistoffen im Körper.

Kompetitiver Immunoassay: Ein kompetitiver Immunoassay wird zur Bestimmung eines Analyten (Antigens) genutzt, wenn für dieses entweder nur ein einzelner spezifischer Antikörper zur Verfügung steht oder wenn der Analyt nicht über ausreichende Bindungsstellen für die ungehinderte Bindung von zwei Antikörpern verfügt. Dabei wird beispielsweise als Erkennungskomponente ein Antikörper (Fängerantikörper) und als kompetitive Komponente ein mit einem Marker markiertes Antigen verwendet.

Sandwich-Assay: Für den Nachweis eines Analyten (Antigens) mittels eines auch als Sandwich-Assay bezeichneten, nicht-kompetitiven Assays werden zwei verschiedene Antikörper benötigt, die den Analyten erkennen und sich dabei nicht in ihrer Bindung an den Analyten gegenseitig behindern. Von Vorteil beim Vergleich mit dem kompetitiven Immunoassay ist insbesondere die bei den meisten Anwendungen höhere Sensitivität.

heterogener Immunoassay: Bei einem heterogenen Immunoassay der gegenständlichen Erfindung findet - im Gegensatz zum homogenen Immunoassay - im Verlauf des Prozesses ein Wechsel der flüssigen Phase statt. Bei Verwendung magnetischer Partikel mit daran gebundenen Fängerantikörpern zur selektiven Bindung des Analyten (Antigens) kann dies z.B. dadurch erreicht werden, dass die Partikel durch ein Magnetfeld an die Gefäßwand abgeschieden werden, die erste Flüssigkeit durch eine zweite Flüssigkeit ersetzt wird, und die Partikel in der zweiten Flüssigkeit resuspendiert werden. Nach dem Entfernen der ersten Flüssigkeit können an den Partikeln beliebige Waschschritte mit der zweiten Flüssigkeit oder einer speziellen Waschflüssigkeit erfolgen. Die Waschschritte ermöglichen die Entfernung von unspezifisch an den Partikeln gebundenen Substanzen sowie von, in der ersten Flüssigkeit vorhandenen störenden Substanzen, wobei durch die Entfernung störender Substanzen der Assay wesentlich spezifischer und empfindlicher wird und niedrige Nachweisgrenzen und Konzentrationsbereiche für das zu bestimmende Antigen erzielt werden.

magnetische Partikel (magnetic beads): Dabei handelt es sich um typischerweise wenige µm große, in einer wässrigen Pufferlösung suspendierte Magnetpartikel, die für immunchemische Tests mit dem Fängerantikörper beschichtet sind.

Fängerantikörper (capture antibody): Das sind Antikörper, die an zumindest ein Epitop des Analyten binden und an der festen Phase, - im Fall der gegenständlichen Erfindung - an der Oberfläche fester magnetischer Partikel gebunden sind.

Tracer-Antikörper (labeled antibody, Konjugat): Dabei handelt es sich um einen zweiten Antikörper, an den chemisch ein Markermolekül (Label) gebunden ist und beim Assay durch Antigen-Antikörper Wechselwirkungen selektiv an Analytmoleküle bindet.

Tracer-Antigen (labeled antigen): Mit Marker gelabeltes Antigen, welches im Falle eines kompetitiven Assays mit dem Analyt um Bindungsstelle an den Fängerantikörpermolekülen konkurriert.

Marker/Markermolekül: Das Markermolekül kann ein Farbstoff sein, der durch Zugabe ein oder mehrerer chemischer Substanzen Licht aussendet (Chemolumineszenz). Weiters kann der Farbstoff auch durch Anlegen einer Spannung (Elektrolumineszenz), oder durch Einstrahlen von Licht (z.B. Fluoreszenz) zur Emission angeregt werden. Das Markermolekül kann auch eine Nachweisreaktion katalysieren, dessen Reaktionsprodukte detektierbar sind, wie beispielsweise im Fall einer enzymatischen Umsetzung (enzymatischer Immunoassay).

Bound/Free-Waschen, bzw. (B/F) - Waschen: Ein Prozessschritt eines heterogenen Immunoassays, bei dem es zur Trennung der spezifisch an der festen Phase gebundenen (bound) und des ungebundenen Rests (free) kommt, wobei letzterer in diesem Waschschritt entfernt wird. Bei dem ungebundenen Rest kann es sich um die ungebundene Matrix der Probe, um den ungebundenen Überschuss an zugegebenen markierten Tracer-Antikörpern (labeled antibody) oder andere überschüssige Bestandteile im Proben-Reagenzien-Gemisch handeln.

Pipettor: eine zum Flüssigkeitstransfer zwischen einem ersten und zweiten Gefäß geeignete Vorrichtung zur Aspiration und anschließendem Ausstoß einer zu transferierenden Flüssigkeit, die verschiedene Bereiche der Proben- und Reagenzienlager, sowie die Küvetten anfahren kann.

Dispensor (bzw. Injektor): Ein Dispensor dient zur Abgabe definierter Flüssigkeitsmengen aus einem Vorratsgefäß über eine Versorgungsleitung die in einer Düse, Abgabeöffnung oder Dispensornadel endet, in ein Gefäß, beispielsweise in eine Küvette.

### Stand der Technik:

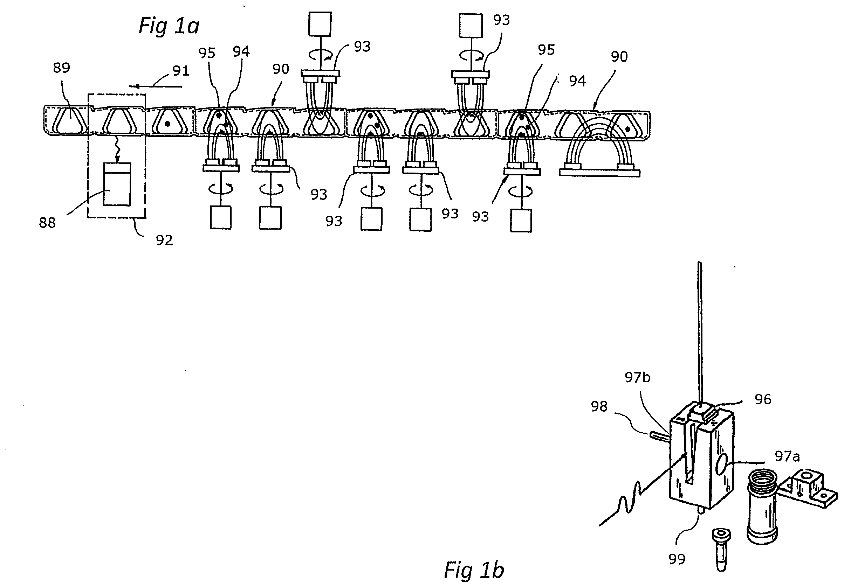

Aus der US 6,333,008 B1 ist eine Messanordnung bekannt, die zur Durchführung luminometrischer Reihenanalysen an Flüssigproben dient, in denen nachzuweisende Zielsubstanzen und mit diesen in einer immunchemischen Nachweisreaktion verbindbare Markiersubstanzen ("labeling substances") sowie magnetisierbare Trägerpartikel enthalten sind. Wie in **Fig. 1a** der gegenständlichen Anmeldung dargestellt, werden die Flüssigproben 89 in Vertiefungen einer Mehrfachküvette 90 entlang einer Förderstrecke (siehe Pfeil 91) zu einer optischen Messstation 92 transportiert, wobei während des Transports als drehbare Doppelmagnete 93 ausgebildete Dauermagnete und Trennstationen, die zur Abtrennung überschüssiger Markiersubstanz bestimmt sind, auf die Mehrfachküvette 90 einwirken. In den einzelnen Trennstationen findet mit Hilfe eines Injektors 94 und einer Saugnadel 95 jeweils ein (B/F) - Waschschritt statt. In der Messstation 92 wird die Lumineszenzstrahlung durch einen Fotodetektor 88 erfasst. Nachteilig bei der bekannten Messanordnung ist die Notwendigkeit, die Flüssigproben während des Analyseprozesses zu verschiedenen, an einem Prozesspfad ortsfest verteilten Automatenkomponenten befördern zu müssen. Weiters müssen bestimmte Komponenten, wie als drehbare Doppelmagnete 93 ausgebildete Dauermagneten und Trennstationen mit Injektoren 94 und Saugnadeln 95 mehrfach ausgebildet werden.

Derartige Vorrichtungen zeichnen sich dadurch aus, dass alle Prozesse durch starre Taktzyklen des Küvetten-Fördermechanismus vorgegeben sind und in vorbestimmten Zeitfenstern ablaufen müssen. Aktionen wie Dispensieren, Mischen, Separieren und Messen können nur dann erfolgen, wenn sich die jeweiligen Küvetten an den Positionen der jeweiligen Gerätekomponenten befinden.

So kann eine Probe (nicht jederzeit, sondern) nur dann in eine leere Küvette dispensiert werden, wenn die leere Küvette an der Position des Probenpipettors vorbeifährt und der Küvetten-Beförderungsmechanismus an dieser Position stoppt. Ein Reagenz oder eine Waschflüssigkeit kann nur dann in eine die Probe enthaltende Küvette dispensiert werden, wenn die betreffende Küvette an der Position des Reagenziendispensors vorbeifährt und der Küvetten-Fördermechanismus an dieser Position stoppt. Analoges gilt für das Rühren von Reaktionsgemischen aus der Probe und den Reagenzien in den Küvetten mit mechanischem Rühren und für die optische Messung an der Position der optischen Messeinrichtung.

Bei abgeschlossenen Messungen kann eine Küvette in nachteiliger Weise nicht sofort gewaschen oder gewechselt, und ein neuer Test gestartet werden. Eine Küvette kann erst dann gewaschen oder gewechselt und somit für einen neuen Test bereit gestellt werden, wenn sich die betreffende Küvette an der Position der Küvettenwaschstation oder des Küvettenwechslers befindet und zu einem fixen Zeitpunkt bzw. einer fixen Zeitdauer ab Testbeginn zu dem/der, entsprechend der konzeptionell starr vorgegebenen Zykluszeiten, an der betreffenden Position ein Waschstop oder ein Küvettenwechsel erfolgt bzw. vorgesehen ist. Dadurch sind alle Küvetten gleich lang "blockiert", unabhängig davon, ob die Messdauer an den jeweiligen Tests kurz oder lang ist.

Bei Vorrichtungen zur Durchführung von heterogenen Immunoassays sind einzelne Prozessschritte eines Assays in einer einzelnen Küvette wie beispielsweise Separation der magnetischen Beads (>10 s), (B/F)-Waschen (einige Sekunden) oder Antigen/Antikörper-Reaktionen bzw. Inkubation (ca. 10-20 min) besonders zeitintensiv, während beispielsweise eine Lumineszenzmessung an der jeweiligen Automatenstation vergleichsweise rasch abläuft (einige Sekunden). Wiederholt sich ein Prozessschritt eines in einer Küvette durchzuführenden Assays, so muss die dafür zuständige Automatenkomponente, entlang des Prozesspfades mehrfach vorhanden sein, um nicht einen vollen Bewegungszyklus des Fördermechanismus abwarten zu müssen.

Herkömmliche Vorrichtungen mit stationär entlang eines zyklischen Bewegungspfades von Station zu Station bewegten Küvetten können ein flexibles Timing von Prozessschritten zur Parallelisierung von Analysen in unterschiedlichen Küvetten nicht ohne Weiteres leisten, da sich die Küvetten auf einem zyklischen Fördermechanismus nur gemeinsam bewegen können und die Abarbeitung von Prozessschritten daher starren Taktzyklen folgt.

Die ortsfeste Verteilung von Automatenkomponenten an einem Prozessweg bei Förderbändern oder Karussellen mit bewegten Proben, Reagenzien und Küvetten, resultiert in verhältnismäßig hohen Durchlaufzeiten für die einzelnen Tests und begrenzt die Anzahl der Tests, die pro Stunde auf einem Gerät mit einer bestimmten Anzahl an Küvetten durchgeführt werden kann.

Aus der US 7,718,072 B2 ist eine Vorrichtung zur Durchführung heterogener Immunoassays bekannt. Wie in **Fig. 1b** der vorliegenden Anmeldung dargestellt, werden die Küvetten 96, welche die Probe, die erforderlichen Reagenzien und magnetische Partikel aufnehmen, in einem Prozessweg mittels Förderband durch eine Reihe von thermostatisierbaren Strukturen hindurchgeführt, wobei jede Struktur eine seitliche Öffnung 97a für den Zugang eines Fotomultipliers, eine gegenüberliegende Freistellung 97b für einen an die Küvette heranführbaren Separationsmagneten 98 sowie einen im Bodenbereich der Küvette 96 angreifenden Mixer 99 aufweist. Mehrere derartige thermostatisierbare Strukturen zur Mischung, magnetischen Separation oder Messung können entlang des Prozessweges stationär angeordnet sein, um die Prozessschritte eines heterogenen Immunoassays in ein oder mehreren Küvetten 96 nacheinander ablaufen zu lassen. Es ist auch hier von Nachteil, dass die an den stationären Automatenkomponenten stattfindenden Prozesse durch starre Taktzyklen des Küvetten-Fördermechanismus vorgegeben sind und in vorbestimmten Zeitfenstern ablaufen müssen.

Aufgabe der Erfindung ist es, ein Verfahren und eine Vorrichtung zur Durchführung von heterogenen Immunoassays ausgehend vom dargelegten Stand der Technik, vorzuschlagen, mit welchem bzw. mit welcher Nachteile, vor allem im Zusammenhang mit dem - durch starre Taktzyklen vorgegeben und in vorbestimmten Zeitfenstern ablaufenden Prozessen - eingeschränkten Probendurchsatz bekannter Systeme vermieden werden und Verbesserungen vorzuschlagen, die den Probendurchsatz erhöhen, ohne die Einzelanalyse oder die Vorrichtung wesentlich zu verteuern, wobei die Qualität der Analyse zumindest beibehalten werden soll.

Diese Aufgabe wird erfindungsgemäß durch eine Vorrichtung zur Durchführung von heterogenen Immunoassays gelöst, die die Merkmale des Anspruchs 1 aufweist.

Die Vorrichtung weist folgende Komponenten auf:
zumindest ein stationäres Küvettenarray, in welchem die Küvetten zur Aufnahme flüssiger Medien (Proben, Reagenzien, Suspensionen, Waschlösungen) angeordnet sind,
zumindest einen, entlang des Küvettenarrays verfahrbaren und in Richtung der Füllöffnung einer ausgewählten Küvette absenkbaren Haltearm mit zumindest einer, in Richtung Boden der Küvette absenkbaren Absaugnadel, sowie mit zumindest einem, über oder in der jeweiligen Füllöffnung positionierbaren Dispensor zur Abgabe der flüssigen Medien in die Küvette, wobei zumindest ein Dispensor zur Abgabe einer Waschlösung für die magnetischen Partikel ausgebildet ist,
zumindest eine, entlang des Küvettenarrays verfahrbare, auf den Inhalt der ausgewählten Küvette wirkende Magnetanordnung zur Separation der magnetischen Partikel an einer Innenfläche der Küvette, sowie
zumindest eine, entlang des Küvettenarrays verfahrbare, und auf das Messfenster der ausgewählten Küvette ausrichtbare, optische Detektionseinrichtung zur Aufnahme eines mit einer Analytkonzentration in der ausgewählten Küvette korrelierenden (beispielsweise direkt oder indirekt proportionalen) Messsignals.

Diese Aufgabe wird auch durch Verfahren gemäß der unabhängigen Verfahrensansprüche gelöst.

Eine erste Variante (Two-Step Assay) des erfindungsgemäßen Verfahrens zur Bestimmung eines Analyten mittels eines heterogenen Immunoassays, wobei in aneinandergereihte Küvetten - jeweils aufweisend eine Füllöffnung und zumindest ein seitliches Messfenster - Proben und magnetische Partikel eingebracht werden, ist dadurch gekennzeichnet, dass zunächst in einer ersten Schrittfolge A
a) eine Probe zur Bestimmung des Analyten, sowie
b) eine Suspension der magnetischen Partikel mit einem Fängerantikörper
in eine ausgewählte Küvette der als stationäres Küvettenarray angeordneten, aneinandergereihten Küvetten einpipettiert werden, sowie dass die folgenden Schritte B einer immunchemischen Analyse, wie
c) Inkubation und Rühren des Küvetteninhalts,
d) Separieren der magnetischen Partikel,
e) ein oder mehrmaliges Einbringen und Absaugen einer Waschlösung,
f) Einpipettieren eines Tracer-Antikörpers oder eines Tracer-Antigens und erneutes Ausführen der Schritte d) und e)
g) Zudosieren zumindest einer Trigger-Flüssigkeit, sowie
h) luminometrische Vermessung der Probe,
mit Hilfe zumindest eines entlang des Küvettenarrays verfahrbaren Mess- und Manipulationsmoduls erfolgen, das zur Durchführung einzelner oder aller Schritte d), e), g) und h) bei der ausgewählten Küvette angehalten wird.

Eine zweite Variante (Single-Step Assay) des erfindungsgemäßen Verfahrens zur Bestimmung eines Analyten mittels eines heterogenen Immunoassays, wobei in aneinandergereihte Küvetten (201) - jeweils aufweisend eine Füllöffnung (207) und zumindest ein seitliches Messfenster (202) - Proben und magnetische Partikel eingebracht werden, ist dadurch gekennzeichnet, dass zunächst in einer ersten Schrittfolge A
a) eine Probe zur Bestimmung des Analyten,
b) eine Suspension der magnetischen Partikel mit einem Fängerantikörper, sowie
c) ein Tracer-Antikörper oder ein Tracer-Antigen
in eine ausgewählte Küvette der als stationäres Küvettenarray angeordneten, aneinandergereihten Küvetten einpipettiert werden, sowie dass die folgenden Schritte B einer immunchemischen Analyse, wie
d) Inkubation und Rühren des Küvetteninhalts,
e) Separieren der magnetischen Partikel,
f) ein oder mehrmaliges Einbringen und Absaugen einer Waschlösung,
g) Zudosieren zumindest einer Trigger-Flüssigkeit, sowie
h) luminometrische Vermessung der Probe,
mit Hilfe zumindest eines entlang des Küvettenarrays verfahrbaren Mess- und Manipulationsmoduls erfolgen, das zur Durchführung einzelner oder aller Schritte e) bis h) bei der ausgewählten Küvette angehalten wird.

Besonders vorteilhaft können insbesondere jene Schritte, die mit dem verfahrbaren Mess- und Manipulationsmodul durchgeführt werden, vollautomatisch erfolgen.

Ein besonderer Vorteil der Erfindung besteht darin, dass das Mess- und Manipulationsmodul während des Ablaufs zeitaufwändiger Schritte bei der immunchemischen Analyse, wie Inkubation, etc., in der ausgewählten Küvette, zu zumindest einer weiteren Küvette des Küvettenarrays verfahren werden kann, um in der weiteren Küvette einzelne oder alle Schritte e) bis h) einer immunchemischen Analyse durchzuführen.

Insbesondere kann das erfindungsgemäße Mess- und Manipulationsmodul frei zwischen den Küvetten des stationären Küvettenarrays verfahren, um während eines nicht mit den Komponenten des Mess- und Manipulationsmoduls durchzuführenden Assay-Prozessschrittes in einer ersten Küvette, einen zweiten Prozessschritt in einer anderen Küvette durchzuführen.

Vor oder während der Anfahrt des Mess- und Manipulationsmoduls zu einer Küvette kann die Nadelgruppe der Dispensoren sowie die Absaugnadel in einer auf dem Mess- und Manipulationsmodul angeordneten Waschstation gewaschen werden.

So kann beispielsweise in einem ersten Parallelisierungsbeispiel während eines Inkubationsschritts eines Assays in einer ersten Küvette eine magnetische Separation sowie (B/F)-Waschen in einer zweiten Küvette durchgeführt werden, um die Auslastung der Automatenkomponenten zu erhöhen, und Zeit bei der Abarbeitung der Assays zu sparen.

Falls eine zusätzliche, verfahrbare Magnetanordnung vorhanden ist, welche unabhängig von den anderen Komponenten des Mess- und Manipulationsmoduls entlang des Küvettenarrays auf einer eigenen Schiene verfahrbar ist, so kann in einem zweiten Parallelisierungsbeispiel eine vollständige magnetische Separierung oder partielle Vorseparierung von Beads eines dritten Assays in einer dritten Küvette durchgeführt werden, während in einem vierten Assay einer vierten Küvette die Prozessschritte der Lumineszenztriggerung und Lumineszenzmessung mithilfe von Komponenten des Mess- und Manipulationsmoduls durchgeführt werden. Die zusätzliche verfahrbare Magnetanordnung kann auf der dem Mess- und Manipulationsmodul gegenüberliegenden Seite des Küvettenarrays angeordnet sein und dort unabhängig von dem Mess- und Manipulationsmodul verfahren werden.

Gemäß einer bevorzugten Ausführungsvariante der Erfindung weist der Haltearm für die Absaugnadel und den zumindest einen Dispensor eine Hub- und Dreheinrichtung auf, die auf einer entlang des Küvettenarrays verfahrbaren Plattform angeordnet ist, wobei auf der verfahrbaren Plattform eine gemeinsame Aufhängung für die Magnetanordnung und die Detektionseinrichtung angeordnet ist.

Besonders vorteilhaft ist es, wenn der auf der verfahrbaren Plattform angeordnete Haltearm samt Dispensorplattform zusammen mit der Magnetanordnung und der Detektionseinrichtung ein entlang des Küvettenarrays verfahrbares Mess- und Manipulationsmodul bildet, das sämtliche robotische, fluidische und messtechnische Komponenten für die Prozessschritte der magnetischen Separation der Beads, des sogenannten (B/F) - Waschens, sowie der Auslösung (Triggerung) und Messung der Lumineszenz, vereint.

Die Erfindung wird im Folgenden an Hand eines Ausführungsbeispiels näher erläutert. Es zeigen:
- Fig. 1a: und Fig. 1b zwei unterschiedliche Vorrichtungen zur Durchführung heterogener Immunoassays gemäß Stand der Technik,
- Fig. 2: eine erfindungsgemäße Vorrichtung zur Durchführung heterogener Immunoassays in einer dreidimensionalen Ansicht,
- Fig. 3: ein Detail der Vorrichtung gemäß Fig. 2 in einer vergrößerten Schnittdarstellung,
- Fig. 4a: ein schematisiertes Ablaufbeispiel eines Two-Step Sandwich-Assays,
- Fig. 4b: ein schematisiertes Ablaufbeispiel eines Single-Step Sandwich-Assays,
- Fig. 4c: ein schematisiertes Ablaufbeispiel eines Two-Step kompetitiven Assays,
- Fig. 4d: ein schematisiertes Ablaufbeispiel eines Single-Step kompetitiven Assays,
- Fig. 5: ein Fluidschaltbild der Vorrichtung gemäß Fig. 2,
- Fig. 6: ein Blockschaltbild zur elektronischen Steuerung der Vorrichtung gemäß Fig. 2, sowie
- Fig. 7: ein Diagramm zum zeitlichen Ablauf der einzelnen Aktivitäten in der erfindungsgemäßen Vorrichtung während eines heterogenen Immunoassays.

Die in den Figuren 1a und 1b dargestellte Vorrichtungen betreffen Beispiele zum Stand der Technik und wurden bereits in der Beschreibungseinleitung ausführlich dargelegt.

Funktionsgleiche Teile sind in den einzelnen Darstellungen der Erfindung mit gleichen Bezugszeichen versehen.

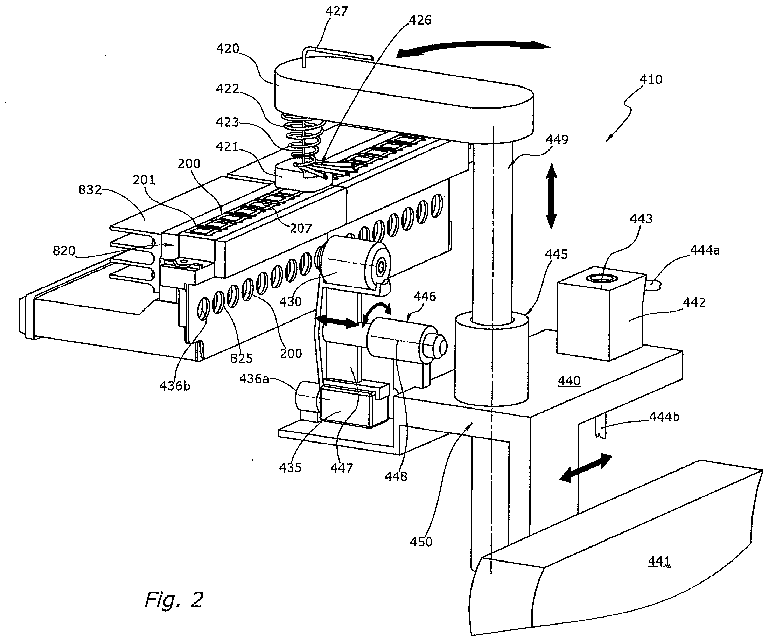

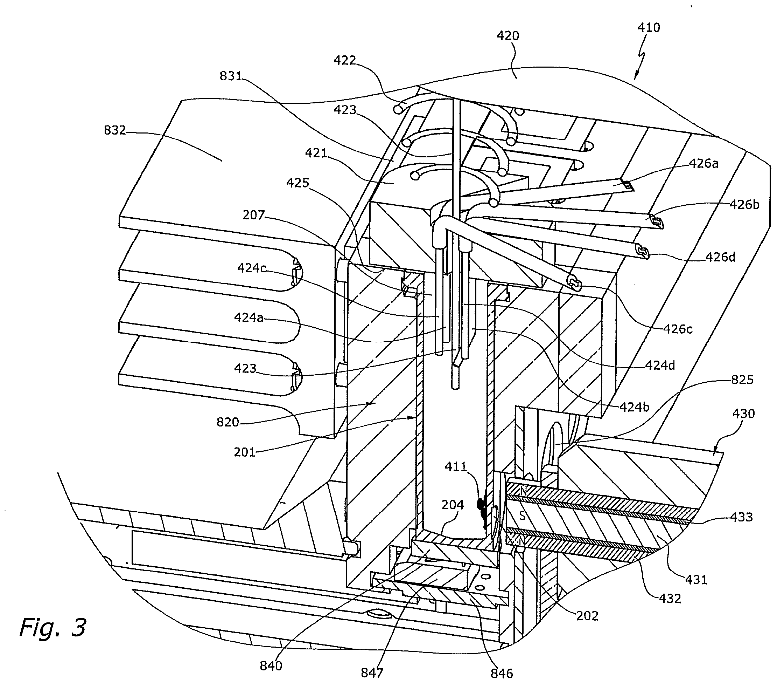

Die in den Fig. 2 und 3 dargestellte, erfindungsgemäße Vorrichtung 410 dient zur Durchführung von heterogenen Immunoassays.

Die Vorrichtung weist ein eindimensionales, stationäres, beispielsweise in einem Analysator angeordnetes Küvettenarray 200 auf, in welchem die Küvetten 201 zur Aufnahme flüssiger Medien (Proben, Reagenzien, Suspensionen mit magnetischen Partikeln 411, Waschlösungen) in einem thermostatisierten Küvettenblock 820 angeordnet sind.

Ein verschwenkbarer Haltearm 420 ist entlang des Küvettenarrays 200 verfahrbar ausgeführt und kann in Richtung der Füllöffnung 207 einer von der Steuerlogik der Vorrichtung ausgewählten Küvette 201 abgesenkt werden. Der Haltearm 420 ist mit einer, in Richtung Boden 204 der Küvette 201 absenkbaren Absaugnadel 423 samt Absaugleitung 427 ausgestattet, sowie mit zumindest einem, über oder in der jeweiligen Füllöffnung 207 positionierbaren Dispensor 424a bis 424d zur Abgabe der flüssigen Medien in die Küvette 201. Zumindest ein Dispensor 424a, 424b ist zur Abgabe einer Waschlösung für die magnetischen Partikel 411 ausgebildet.

Die Zuleitungen zu den Dispensoren 424a, 424b sind mit 426 bezeichnet, im Speziellen führt eine Waschleitung 426a zum Dispensor 424a, eine Waschleitung 426b zum Dispensor 424b, eine Zuleitung 426c zum Dispensor für eine Pretriggerlösung und eine Zuleitung 426d zum Dispensor 424d für eine Triggerlösung.

Weiterhin ist eine, entlang des Küvettenarrays 200 verfahrbare, auf den Inhalt der ausgewählten Küvette 201 wirkende Magnetanordnung 430 zur Separation der magnetischen Partikel 411 an einer Innenfläche der Küvette 201 vorgesehen, sowie eine, entlang des Küvettenarrays 200 verfahrbare optische Detektionseinrichtung 435, die auf das Messfenster 202 der ausgewählten Küvette 201 ausgerichtet werden kann, um ein mit der Analytkonzentration in der ausgewählten Küvette 201 korrelierendes (z.B. direkt oder indirekt proportionales) Messsignal zu erhalten.

Zur Vereinfachung sind nur jene Komponenten der Vorrichtung 410 dargestellt, die für die gegenständliche Erfindung wesentlich sind, wobei auf Analysatorkomponenten, wie Proben- und Reagenzienlager, Pumpen, Ventile, Auswerte-, Steuer- und Antriebseinheiten, nicht näher eingegangen wird.

Das Küvettenarray 200 ist in einem thermostatisierbaren Küvettenblock 820 angeordnet, wobei insbesondere in Fig. 3 die für die Thermostatisierung vorgesehenen Peltier-Elemente 831 erkennbar sind, die zwischen Kühlrippen 832 und dem Küvettenblock 820 angeordnet sind. Der Küvettenblock 820 weist an der Vorderseite mit den Messfenstern 202 der Küvetten 201 fluchtende Zugangsöffnungen 825 auf. Zum Mischen und Thermostatisieren des Küvetteninhalts ist am Boden 204 jeder Küvette 201 ein Ultraschall-Wandler 840 (z.B. piezoelektrischer Dickenschwinger) befestigt, wobei der Kontakt zu einer Federkontaktplatine 846 über Kontaktblöcke 847 mit Federkontakten hergestellt wird.

Alternativ dazu kann der Küvetteninhalt beispielsweise durch die Absaugnadel 423, oder durch einen zum Einbringen der Probe und/oder Reagenzien genutzten manuellen oder automatischen Pipettor gemischt werden, indem die Flüssigkeit einer Küvette 201 durch wiederholtes Ansaugen und Ausstoßen zumindest eines Teils des Flüssigkeitsvolumens, Einbringung der Proben und/oder Reagenzien mit hoher Strömungsgeschwindigkeit bzw. einer geeigneten Kombination dieser Maßnahmen homogenisiert wird.

Weitere alternative Vorrichtungen zum Mischen des Küvetteninhalts können das Eintauchen einer Rührwelle in die Küvette, sowie das Rühren durch eine horizontale Orbitalbewegung des Küvettenblocks 820 oder durch eine in die Küvette eintauchende Pipettiernadel umfassen.

Am verfahrbaren Haltearm 420 ist an einer federnden Halterung (siehe Federelement 422) eine auf die Füllöffnung 207 der Küvette 201 absenkbare Dispensorplattform 421 befestigt, die im dargestellten Beispiel vier Dispensoren 424a bis 424d zur Abgabe flüssiger Medien in die Küvette 201 aufweist. Die Dispensorplattform 421 wird in einer zentralen Öffnung von der am Haltearm 420 befestigten Absaugnadel 423 durchsetzt, sodass diese nach Anlage der Dispensorplattform 421 an der Füllöffnung 207 der Küvette 201 bis zum Boden 204 der Küvette 201 abgesenkt werden kann.

Die Dispensorplattform 421 weist an der der Küvette 201 zugewandten Seite eine Dichtfläche 425 oder eine formschlüssige Abschottung aus einem lichtundurchlässigen Material auf, sodass bei abgesenkter Dispensorplattform 412 der Zutritt von Umgebungslicht bei der optischen Vermessung des Küvetteninhalts ausgeschlossen ist.

Erfindungsgemäß weist ein Dispensor 424a zur Abgabe einer Waschlösung für die magnetischen Partikel 411 eine im Wesentlichen parallel zur Längsachse der Küvette 201 ausgerichtete Ausströmungsrichtung (gerade Waschnadel) auf, und ein zweiter Dispensor 424b - ebenfalls zur Abgabe einer Waschlösung - eine auf eine innere Seitenfläche der Küvette 201 gerichtete Ausströmungsrichtung (schräge Waschnadel) auf.

Von zwei weiteren Dispensoren 424c, 424d der Dispensorplattform 412, deren Ausströmungsrichtungen im Wesentlichen parallel zur Längsachse der Küvette 201 ausgerichtet sind, ist ggf. ein dritter Dispensor 424c zur Abgabe einer Pretriggerlösung und ein vierter Dispensor 424d zur Abgabe einer Triggerlösung ausgebildet. Für auf Chemolumineszenz basierende Immunoassays, die mit nur einer Triggerlösung auskommen, kann der dritte Dispensor 424c ungenutzt bleiben oder entfallen.

Das in den Fig. 2 und 3 dargestellte Ausführungsbeispiel zeichnet sich durch eine entlang des Küvettenarrays 200 verfahrbare Plattform 440 aus, welche eine Hub- und Dreheinrichtung 445 aufweist, mit welcher der Haltearm 420 samt Absaugnadel 423 und den Dispensoren 424a bis 424d der Dispensorplattform 421 absenkbar ausgeführt ist. Bevorzugt ist auf der verfahrbaren Plattform 440 auch eine gemeinsame Aufhängung 446 für die Magnetanordnung 430 und die Detektionseinrichtung 435 angeordnet, sodass ein verfahrbares Mess- und Manipulationsmodul 450 realisiert wird, das sämtliche robotische, fluidische und messtechnische Komponenten für die Prozessschritte der magnetischen Separation der Beads, des sogenannten (B/F) - Waschens, sowie der Auslösung (Triggerung) und Messung der Lumineszenz vereint.

Die verfahrbare Plattform 440 des Mess- und Manipulationsmoduls 450 ist über eine parallel zum Küvettenarray 200 verlaufende, seitliche Schiene 441 mit dem Rahmen der Vorrichtung 410 verbunden, und kann über einen Verfahrmechanismus wie beispielsweise einem schrittmotorangetriebenen Zahnriemen, einer Spindel oder einen Linearmotor auf die Position einer ausgewählten Küvette 201 gebracht werden. Zur Versorgung und Steuerung des Mess- und Manipulationsmoduls 450 können hierbei flexible elektrische und fluidische Verbindungsleitungen beispielsweise in Form sog. Energieketten (nicht dargestellt) an die Plattform 440 herangeführt werden.

Gemäß einer Ausführungsvariante kann auf der verfahrbaren Plattform 440 auch eine Waschstation 442 für die Absaugnadel 423 und den zumindest einen Dispensor 424a bis 424d der Dispensorplattform 421 angeordnet sein, auf deren Öffnung 443 der Haltearm 420 nach einer Drehbewegung absenkbar ausgeführt ist, sodass die gesamte Nadelgruppe am Kopf des schwenkbaren Haltearms 420 in die Öffnung 443 eingeführt werden kann.

Die Nadelwaschstation 442 weist eine den Füllstand begrenzende, obere Absaugleitung 444a und eine untere Absaugleitung 444b auf. Hierbei ist ein Zufahren auf die Öffnung 443 durch eine Auf- und Abbewegung mit einer 90° Drehung bei gleichzeitiger Absenkung des Haltearms 420 unter die Oberkante des Küvettenarrays 200 möglich, wodurch andere Robotikkomponenten, beispielsweise allfällige Pipettoren, etc., ungehindert entlang des Küvettenarrays 200 verfahren können.

Der schwenkbare Haltearm 420 des Mess- und Manipulationsmoduls 450 ist an einem in horizontaler Ebene um 90° schwenkbaren und zusätzlich vertikal beweglichen Turm 449 befestigt, wobei die Schwenkbewegung durch einen beispielsweise schrittmotorangetriebenen Drehaktuator ermöglicht wird. Zusätzlich ist der Turm mit einer Hebevorrichtung ausgestattet, die beispielsweise eine schrittmotorangetriebene Spindel oder einen Zahnriemen zur Erzeugung einer vertikalen Translationsbewegung des Haltearms 420 umfasst. Die beiden Bewegungsarten können in die kombinierte Hub- und Dreheinrichtung 445 an der Basis des vertikalen Turms 449 integriert sein.

Eine Ausführungsvariante kann auch darin bestehen, dass die Nadelwaschstation stationär am Ende des Küvettenarrays 200 positioniert ist, wobei der Haltearm der Nadelgruppe in dieser Variante nicht schwenkbar ausgeführt sein muss.

Gemäß einer bevorzugten Ausführungsvariante ist die gemeinsame Aufhängung 446 für die Magnetanordnung 430 und die Detektionseinrichtung 435 geeignet, eine translatorische oder rotatorische Bewegung auszuführen, um die Positionen der Magnetanordnung 430 und der Detektionseinrichtung 435 vor der ausgewählten Küvette 201 auszutauschen.

Beispielsweise können die Magnetanordnung 430 und die Detektionseinrichtung 435 im gleichen Abstand zu einer gemeinsamen Drehachse 448 an einem in der Aufhängung 446 gelagerten Rotorarm 447 befestigt sein.

Bevorzugt kann dabei der in der Aufhängung 446 gelagerte Rotorarm 447 translatorisch in Richtung der Drehachse 448 verschiebbar ausgeführt sein, um die Magnetanordnung 430 oder die Detektionseinrichtung 435 an die Zugangsöffnung 825 im Küvettenblock 820 und somit an das Messfenster 202 der ausgewählten Küvette 201 heranzuführen. Der Fotomultiplier 435 sowie die Magnetanordnung 430 können mit ihrer jeweiligen optischen Haupt- bzw. Polachse auf die entsprechende Zugangsöffnung 825 im Küvettenblock ausgerichtet werden und durch eine Horizontalbewegung an die jeweilige Öffnung lichtdicht andocken, bzw. zur Erzeugung einer möglichst hohen magnetischen Flussdichte optimal an die Wand der Küvette 201 angenähert werden.

Die Magnetanordnung 430 kann aus einem oder mehreren Magneten bestehen, die vorzugsweise Seltenerdemagneten hoher Feldstärke, wie z.B. Nd₂Fe₁₄B (Neodym-Eisenborat) sind, kann aber auch als Elektromagnet ausgeführt sein. Die Magnetanordnung 430 ist vorzugsweise aus Neodym-Stabmagneten mit zwei verschiedenen Stabradien ausgeführt, wobei im Wesentlichen ein innerer Stab 431 unter Zwischenlage einer nichtmagnetischen Zwischenschicht 433 von einem äußeren, hohlzylindrischen Stab 432 umfasst wird und die zwei Stäbe unterschiedlicher Länge und Durchmesser einen konischen Übergang aufweisen. Die Anordnung läuft in einen schlanken Endbereich mit punktuell hoher magnetischer Flussdichte aus, der durch die Öffnung 825 im Küvettenblock 820 nahe an das Fenster 202 der Küvette 201 herangebracht werden kann. Die Magnetanordnung 430 kann auch aus mehreren, einzelnen Magneten zusammengesetzt sein, um die für die magnetische Separation an einer Küvettenwand notwendige magnetische Feldstärke bzw. den magnetischen Feldgradienten zu erhöhen, oder Streufelder in die Nachbarküvetten zu verringern. Ein Beispiel einer Magnetanordnung ist in Fig. 3 dargestellt, wobei ein bipolares Ende einer konzentrischen Magnetanordnung 430 mit einer nichtmagnetischen Zwischenschicht 433 auf die Küvette 201 gerichtet ist.

Gemäß einer Ausführungsvariante kann die Vorrichtung eine zweite, auf einer separaten Schiene entlang des Küvettenarrays 200 verfahrbare, auf den Inhalt einer ausgewählten Küvette 201 wirkende Magnetanordnung (nicht dargestellt) aufweisen, um von der anderen Seite des Küvettenblocks 820 auf die Küvetten 201 einzuwirken. Hierbei ist eine zur ersten Zugangsöffnung 825 der Küvetten 201 vergleichbare zweite Öffnung nicht unbedingt erforderlich, da die magnetischen Feldlinien der zweiten Magnetanordnung durch das nicht ferromagnetische Material des Küvettenblocks (Aluminium) hindurchwirken können. Mit der zweiten Magnetanordnung können einzelne Prozessschritte übernommen oder vorbereitet werden und so der Gesamtdurchsatz der Vorrichtung erhöht werden. Beispielsweise kann zeitgleich eine magnetische Separation an einer anderen Küvette durchgeführt werden, um magnetische Beads für einen Waschschritt eines zweiten Assays in der anderen Küvette zu separieren. Mit dem Mess- und Manipulationsmodul 450 können danach zeitsparend die erforderlichen Waschschritte durchgeführt werden.

Die zweite Magnetanordnung kann sowohl aus einem oder mehreren Elektromagneten, als auch aus Permanentmagneten bestehen, wobei bei Permanentmagneten ein Aktuator vorgesehen werden muss, um die Magnetanordnung zur Küvette wahlweise anzunähern, oder zu entfernen. Der Aktuatormechanismus kann analog zu dem für die erste Magnetanordnung 430 ausgeführt sein und in bekannter Weise über einen Riemenantrieb, eine Antriebsspindel oder ein Solenoid verfügen.

Die Detektionseinrichtung 435 ist vorzugsweise durch einen kompakt bauenden Fotomultiplier realisiert und dient zur Messung der Lichtmenge während der durch Zugabe der beiden Triggerlösungen ausgelösten Chemolumineszenz, und kann mit einer Peltier-Kühlung ausgestattet sein, um ein konstanteres, rauschärmeres Signal zu erhalten. Zur Vermeidung von Fehllicht bei der Messung an einer der Zugangsöffnungen 825 des Küvettenblocks 820, können die Zugangsöffnungen 825 und die Lichteinlassöffnung des Fotomultipliers konzentrisch gestufte Kontaktflächen am Rand der beiden Öffnungen aufweisen. Weiters kann ein beispielsweise mechanisch betätigtes Blendenelement (Shutter) vorgesehen sein, um den Fotomultiplier im Ruhezustand vor dem Eintritt von Umgebungslicht zu schützen.

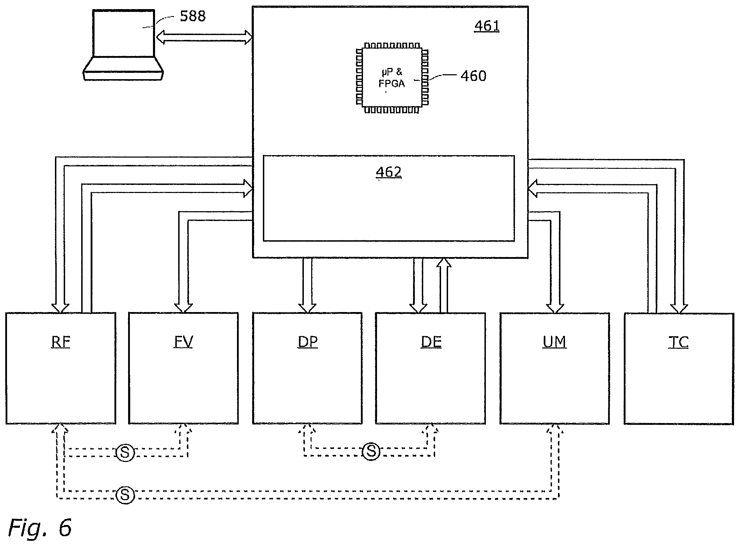

Zur Messung der Lumineszenz bei niedriger Analytkonzentration wird bevorzugt ein digitaler Fotomultiplier eingesetzt, der pro eintreffendes Photon triggert und einen digitalen Impuls von 10 ns auslöst. Diese kurzen Pulse werden mit dem FPGA des HetIA Controllers 460 (siehe Fig. 6) gezählt und als Zählerstand über eine einstellbare Abtastzeit aufsummiert. Solange die Photonenanzahl klein ist, können die unregelmäßig entstehenden Impulse einzeln ausgegeben werden, die Impulsanzahl pro Zeiteinheit entspricht dann der Photonenanzahl pro Zeiteinheit.

Erfindungsgemäß kann auf der verfahrbaren Plattform 440 eine Referenzlichtquelle 436a für die Detektionseinrichtung 435 angeordnet sein. Die Referenzlichtquelle 436a dient zur Kalibration des Fotomultipliers und weist eine Lichtaustrittsöffnung auf, die in Richtung der Eintrittsöffnung der Detektionseinrichtung 435 (z.B. Fotomultiplier) ausgerichtet ist. Die Referenzlichtquelle 436a kann an beliebiger Stelle entlang der Bewegungslinie der Detektionseinrichtung 435 angeordnet sein, idealerweise jedoch derart, dass eine Kalibration des Fotomultipliers dann erfolgt, wenn sich die Magnetanordnung 430 gerade vor der jeweiligen Zugangsöffnung 825 des Küvettenblocks 820 befindet.

Alternativ zu dieser Variante kann eine Referenzlichtquelle 436b auch ortsfest am Ende des Küvettenblocks 820 angeordnet sein und eine Lichtaustrittsöffnung entlang der Zugangsöffnungen des Küvettenblocks 820 aufweisen, wodurch dessen Thermostatisiereinrichtung für die Referenzlichtquelle 436b mitgenutzt werden kann.

Neben einer **Variante 1,** die weiter vorne beschrieben wurde, bei welcher alle vier Dispensoren 424a bis 424d der Dispensorplattform 421 mit deren Nadelspitzen gleichzeitig in die Küvette 201 abgesenkt werden, sind noch weitere Varianten denkbar.

**Variante 2:** Der vertikal bewegliche und verdrehbare Turm 449 kann mehrere Haltearme 420 aufweisen (nicht dargestellt).

Zur Absenkung einzelner Dispensoren 424a bis 424d oder von Dispensorpaaren 424a/424b bzw. 424c/424d in die Küvette 201 kann der Turm 449 so ausgeführt sein, dass er mehrere (bis zu vier) radial am oberen Ende angebrachte Haltearme 420 aufweist. Zwei Haltearme 420 können beispielsweise im Winkel von 90° oder gegenüberliegend, im Winkel von 180° angeordnet sein. An jedem freien Ende der Haltearme 420 befindet sich eine mit der Dispensorplattform 421 vergleichbare Plattform, mit je einer oder zwei der Dispensoren 424a bis 424d und bevorzugt je einer absenkbaren Absaugnadel 423. Der Turm 449 kann im Querschnitt in mehrere, vertikal unabhängig bewegbare Segmente aufgeteilt sein, die jeweils einen Haltearm 420 tragen, sodass es möglich ist, mit Hilfe der Hub- und Dreheinrichtung 445 nur jenes Segment abzusenken, dessen Haltearm sich über einer Küvette 201 befindet

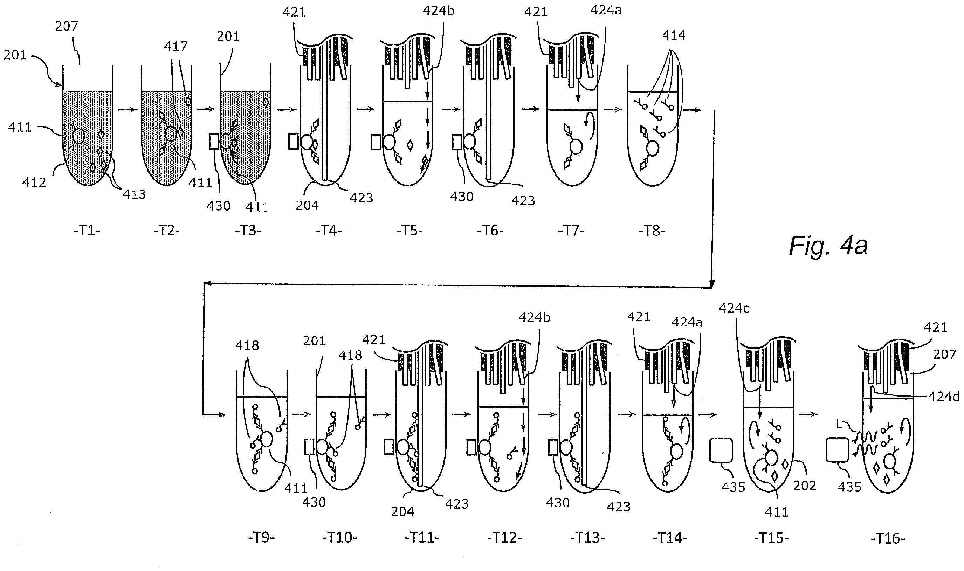

Mehrere Ablaufbeispiele heterogener Immunoassays werden beispielhaft in den Fig. 4a bis 4d dargestellt.

Die vorliegenden Beispiele heterogener Immunoassays beziehen sich auf die erforderlichen maschinellen Prozesse zur automatisierten erfindungsgemäßen Durchführung sogenannter "Sandwich-Assays" und "Kompetitiver Assays", die beide jeweils als "one-step" Assays oder "two-step" Assays ausgeführt werden können.

Beim Sandwich Assay mit Chemolumineszenz-Messung bildet das Analytmolekül durch Antigen-Antikörper-Interaktionen eine Brücke zwischen einem auf einem Träger, beispielsweise der Oberfläche der magnetischen Partikel immobilisierten, ersten Antikörper (Fängerantikörper) und einem zweiten Antikörper, an welchen Signalmoleküle gebunden sind (Tracer-Antikörper), die nach Zugabe einer Pretriggerflüssigkeit und einer Triggerflüssigkeit eine der Analytmenge proportionale, wenige Sekunden anhaltende Chemolumineszenz hervorruft. Die beiden Antikörper-Typen liegen im Vergleich zum Analyten im Überschuss vor. Bei Analytmolekülen, die zu klein sind, um Bindungsstellen für zwei verschiedene Antikörper aufzuweisen, werden sogenannte kompetitive Immunoassays eingesetzt, wobei die Tracer-Antikörper direkt mit den Analytmolekülen um Bindungsstellen an einem, auf einem Träger immobilisierten Antikörper konkurrieren.

### Two-Step Sandwich-Assay

Bei einem Two-Step Sandwich-Assay gemäß Fig. 4a werden zunächst die Probe (enthält den Analyt 413) und eine Suspension magnetischer Partikel 411 (Magnetic Beads) mit einer Beschichtung aus einem Fängerantikörper 412 in die Küvette 201 mittels eines hier nicht dargestellten Pipettors einpipettiert (T1, in Fig. 4a).

Während der nachfolgenden Inkubation (ca. 3 - 20 min) bei 37 °C, wird die Lösung beispielsweise mittels Ultraschall gemischt, um ein Absinken und Agglomerieren der Partikel zu verhindern und um eine gute Durchmischung aller Komponenten zu gewährleisten. Nach der Inkubation sind die Analytmoleküle 413 an den auf den Partikeln 411 immobilisierten Fängerantikörpern 412 gebunden. Weiters gibt es unspezifisch gebundene Analytmoleküle 417 an der Partikeloberfläche und/oder der Küvettenwand. (T2, in Fig. 4a).

Die Partikel 411 samt den daran gebundenen Substanzen werden mit Hilfe der Magnetanordnung 430 an der Innenwand der Küvette 201 fixiert (T3, in Fig. 4a).

Nach der Fixierung der Partikel wird die gesamte Flüssigkeit weitgehend mit der aus der Dispensorplattform 421 abgesenkten Absaugnadel 423 entfernt (T4, in Fig. 4a).

In weiterer Folge wird eine Waschlösung durch eine schräg auf die Innenwand der Küvette 201 gerichtete Dispensornadel 424b eingebracht, um an den Partikeln 411 und/oder der Innenwand der Küvette verbliebene bzw. unspezifisch anhaftende überschüssige Bestandteile durch vorsichtige Spülung zu entfernen, wobei die Partikel 411 nach wie vor magnetisch an der Gefäßwand gehalten werden (T5, in Fig. 4a).

Anschließend wird die Küvette 201 erneut trocken gesaugt, wobei die Partikel 411 samt den daran gebundenen Substanzen noch immer magnetisch an der Innenwand der Küvette 201 fixiert sind (T6, in Fig. 4a).

Eine zweite, vertikal ausgerichtete Dispensornadel 424a hingegen erzeugt beim Eindüsen von Waschlösung oder Verdünnungsflüssigkeit Turbulenzen in der Flüssigkeit, sodass die Partikel 411 bei abgedockten Magneten in der Flüssigkeit resuspendiert werden (T7, in Fig. 4a).

Nach den Waschschritten (T4-T7), wird nach einer abschließenden Absaugung eine Lösung des Tracer-Antikörpers 414 in die Küvette 201 mittels eines hier nicht dargestellten Pipettors einpipettiert (T8, in Fig. 4a).

Während der anschließenden Inkubation (ca. 3 - 6 min) bei 37 °C, wird die Lösung beispielsweise mittels Ultraschall gemischt, um ein Absinken und Agglomerieren der Partikel 411 zu verhindern und um eine gute Durchmischung aller Komponenten zu gewährleisten. Am Ende der Inkubationsphase sind die Analytmoleküle 413 "sandwichartig" zwischen dem auf den Partikeln 411 immobilisierten Fängerantikörper 412 und dem zugegebenen Tracer-Antikörper 414 gebunden.

Weiters gibt es unspezifisch gebundene Tracer-Antikörper 418 an der Partikeloberfläche und/oder der Küvettenwand (T9, in Fig. 4a).

Die Partikel 411 samt den daran gebundenen Substanzen werden nun mit Hilfe der Magnetanordnung 430 an der Innenwand der Küvette 201 fixiert (T10, in Fig. 4a)

Nach Fixierung der Partikel 411 wird die gesamte Flüssigkeit weitgehend, mit der aus der Dispensorplattform 421 abgesenkten Absaugnadel 423 entfernt (T11, in Fig. 4a).

In weiterer Folge wird eine Waschlösung durch eine schräg auf die Innenwand der Küvette 201 gerichtete Dispensornadel 424b eingebracht, um an den Partikeln 411 und/oder der Innenwand der Küvette verbliebene bzw. unspezifisch anhaftende überschüssige Bestandteile durch vorsichtige Spülung der Partikel 411 zu entfernen, wobei die Partikel 411 nach wie vor magnetisch an der Gefäßwand gehalten werden (T12, in Fig. 4a).

Anschließend wird die Küvette 201 erneut trocken gesaugt, wobei die Partikel 411 samt den daran gebundenen Substanzen noch immer magnetisch an der Innenwand der Küvetten 201 fixiert sind (T13, in Fig. 4a).

Eine zweite, vertikal ausgerichtete Dispensornadel 424a hingegen erzeugt beim Eindüsen von Waschlösung oder Verdünnungsflüssigkeit Turbulenzen in der Flüssigkeit, sodass die Partikel 411 bei abgedockten Magneten in der Flüssigkeit resuspendiert werden (T14, in Fig. 4a).

Nach diesen Waschschritten (T11-T14), wird nach einer abschließenden magnetischen Separation und Absaugung (nicht dargestellt) der Fotomultiplier 435 an die Küvette 201 herangefahren. Durch die beiden Dispensoren 424c und 424d werden nun zunächst Pretrigger- (T15, in Fig. 4a) und nach einer Einwirkzeit (ca. 5 Sekunden) Triggerlösung (T16, in Fig. 4a) zugeführt. Damit wird eine nur wenige Sekunden andauernde Chemilumineszenz L (Flash Luminescence) ausgelöst, die vom Fotomultiplier 435 gemessen wird. Die hierfür auf die Füllöffnung 207 der Küvette 201 aufgesetzte Dispensorplattform 421 des Haltearms sorgt gleichzeitig für die hierzu benötigte Verdunkelung der Küvette 201.

### Single-Step Sandwich-Assay

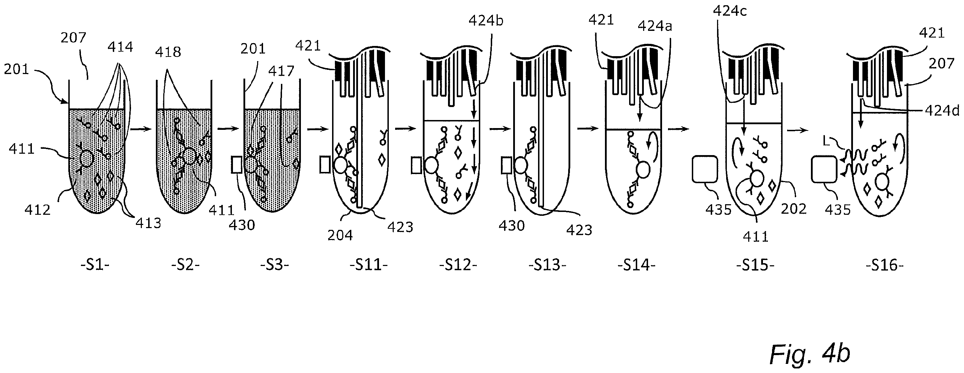

Bei einem Single-Step Sandwich-Assay gemäß Fig. 4b werden zunächst die Probe (enthält den Analyt 413), eine Suspension magnetischer Partikel 411 mit einer Beschichtung aus einem Fängerantikörper 412, sowie eine Lösung des Tracer-Antikörpers 414 in die Küvette 201 mittels eines hier nicht dargestellten Pipettors einpipettiert (S1, in Fig. 4b).

Während der nachfolgenden Inkubation (ca. 10 min) bei 37 °C wird die Lösung beispielsweise mittels Ultraschalles gerührt, um ein Absinken und Agglomerieren der Beads zu verhindern und um eine gute Durchmischung aller Komponenten zu gewährleisten. Nach der Inkubation sind die Analytmoleküle 413 "sandwichartig" zwischen einem auf den Partikeln 411 immobilisierten Fängerantikörper 412 und einem Tracer-Antikörper 414 gebunden. Weiters gibt es unspezifisch gebundene Analytmoleküle 417 und Tracer-Antikörper 418 an der Partikeloberfläche und/oder der Küvettenwand (S2, S3 in Fig. 4b).

Die Partikel 411 samt den daran gebundenen Substanzen werden mit Hilfe der Magnetanordnung 430 an der Innenwand der Küvette 201 fixiert (S3, in Fig. 4b)

Die den Schritten T4 bis T10 gemäß Fig. 4a entsprechenden Schritte entfallen beim Single-Step Sandwich-Assay.

Nach der Fixierung der Partikel wird die gesamte Flüssigkeit weitgehend mit der aus der Dispensorplattform 421 abgesenkten Absaugnadel 423 entfernt (S11, in Fig. 4b).

In weiterer Folge wird eine Waschlösung durch eine schräg auf die Innenwand der Küvette 201 gerichtete Dispensiernadel 424b eingebracht, um an den Partikeln 411 und/oder der Innenwand der Küvette verbliebene bzw. unspezifisch anhaftende überschüssige Bestandteile durch vorsichtige Spülung der Partikel zu entfernen, wobei die Partikel 411 nach wie vor magnetisch an der Gefäßwand gehalten werden (S12, in Fig. 4a).

Anschließend wird die Küvette 201 erneut trocken gesaugt, wobei die Partikel 411 samt den daran gebundenen Substanzen noch immer magnetisch an der Innenwand der Küvetten 201 fixiert sind (S13, in Fig. 4b).

Eine zweite, vertikal ausgerichtete Dispensiernadel 424a hingegen erzeugt beim Eindüsen von Waschlösung oder Verdünnungsflüssigkeit Turbulenzen in der Flüssigkeit, sodass die Partikel 411 bei abgedockten Magneten in der Flüssigkeit resuspendiert werden (S14, in Fig. 4b).

Nach den Waschschritten S11-S14 wird nach einer abschließenden magnetischen Separation und Absaugung (nicht dargestellt) der Fotomultiplier 435 an die Küvette 201 herangefahren. Durch die beiden Dispensoren 424c und 424d werden nun zunächst Pretriggerlösung (S15, in Fig. 4b) und nach einer Einwirkzeit (ca. 5 Sekunden) Triggerlösung (S16, in Fig. 4b) zugeführt. Damit wird eine nur wenige Sekunden andauernde Chemolumineszenz L (Flash Luminescence) ausgelöst, die vom Fotomultiplier 435 gemessen wird. Die hierfür auf die Füllöffnung 207 der Küvette 201 aufgesetzte Dispensorplattform 421 des Haltearms sorgt gleichzeitig für die hierzu benötigte Verdunkelung der Küvette 201.

### Two-Step kompetitiver Assay

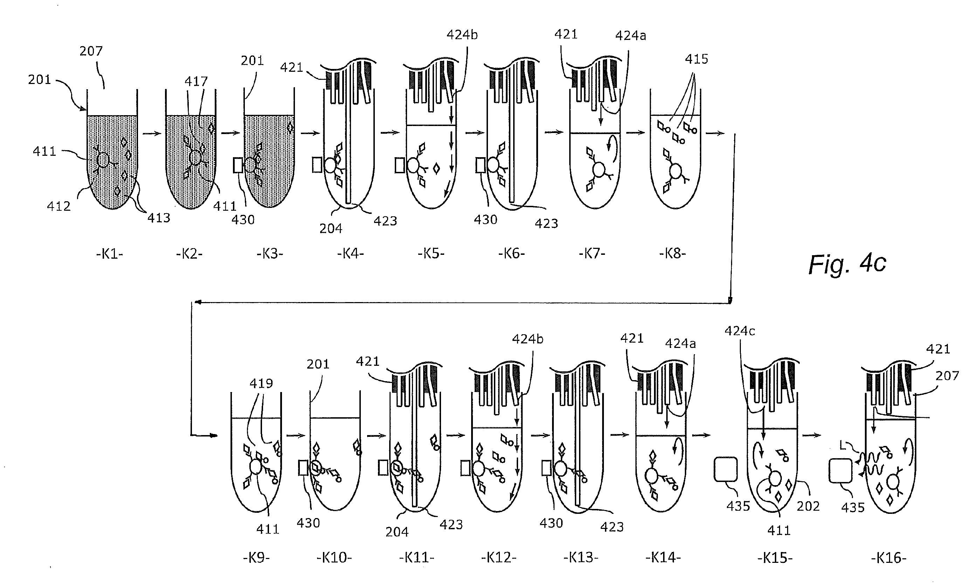

Beim Two-Step kompetitiven Assay gemäß Fig. 4c werden zunächst die Probe (enthält den Analyt 413) und eine Suspension magnetischer Partikel 411 mit einer Beschichtung aus einem Fängerantikörper 412 in die Küvette 201 mittels eines hier nicht dargestellten Pipettors einpipettiert (K1, in Fig.4c).

Während der anschließenden Inkubation (ca. 3 - 20 min) bei 37 °C, wird die Lösung beispielsweise mittels Ultraschall gerührt, um ein Absinken und Agglomerieren der Partikel zu verhindern und um eine gute Durchmischung aller Komponenten zu gewährleisten. Nach der Inkubation sind die Analytmoleküle auf den auf den Partikeln 411 immobilisierten Fängerantikörpern 412 gebunden. Weiters gibt es unspezifisch gebundene Analytmoleküle 417 an der Partikeloberfläche und/oder der Küvettenwand. (K2, in Fig. 4c).

Die Partikel 411 samt den daran gebundenen Substanzen werden mit Hilfe der Magnetanordnung 430 an der Innenwand der Küvette 201 fixiert (K3, in Fig. 4c)

Nach der Fixierung der Partikel wird die gesamte Flüssigkeit weitgehend mit der aus der Dispensorplattform 421 abgesenkten Absaugnadel 423 entfernt (K4, in Fig. 4c).

In weiterer Folge wird eine Waschlösung durch eine schräg auf die Innenwand der Küvette 201 gerichtete Dispensiernadel 424b eingebracht, um an den Partikeln 411 und/oder der Innenwand der Küvette verbliebene bzw. unspezifisch anhaftende überschüssige Bestandteile durch vorsichtige Spülung der Partikel 411 zu entfernen, wobei die Partikel 411 nach wie vor magnetisch an der Gefäßwand gehalten werden (K5, in Fig. 4c).

Anschließend wird die Küvette 201 erneut trocken gesaugt, wobei die Partikel 411 samt den daran gebundenen Substanzen noch immer magnetisch an der Innenwand der Küvetten 201 fixiert sind (K6, in Fig. 4c).

Eine zweite, vertikal ausgerichtete Dispensiernadel 424a hingegen erzeugt beim Eindüsen von Waschlösung oder Verdünnungsflüssigkeit Turbulenzen in der Flüssigkeit, sodass die Partikel 411 bei abgedockten Magneten in der Flüssigkeit resuspendiert werden (K7, in Fig. 4c).

Nach den Waschschritten (K4-K7), wird nach einer abschließenden magnetischen Separation und Absaugung (nicht dargestellt) eine Lösung Tracer-Antigens 415 in die Küvette 201 mittels eines hier nicht dargestellten Pipettors einpipettiert (K8, in Fig. 4c).

Zu Beginn der anschließenden Inkubation (ca. 3 - 6 min) bei 37 °C, wird die Lösung beispielsweise mittels Ultraschall gerührt, um ein Absinken und Agglomerieren der Partikel 411 zu verhindern und um eine gute Durchmischung aller Komponenten zu gewährleisten. Nun sind zusätzlich zu den Analytmolekülen 413 auch die Tracer-Antigenmoleküle 415 auf den verbliebenen Bindungsstellen der auf den Partikeln 411 immobilisierten Fängerantikörper 412 gebunden. Weiters gibt es unspezifisch gebundene Tracer-Antigene 419 an der Partikeloberfläche und/oder der Küvettenwand (K9, in Fig. 4c).

Die Partikel 411 samt den daran gebundenen Substanzen werden nun mit Hilfe der Magnetanordnung 430 an der Innenwand der Küvette 201 fixiert (K10, in Fig. 4c)

Nach Fixierung der Partikel wird die gesamte Flüssigkeit weitgehend mit der aus der Dispensorplattform 421 abgesenkten Absaugnadel 423 entfernt (K11, in Fig. 4c).

In weiterer Folge wird eine Waschlösung durch eine schräg auf die Innenwand der Küvette 201 gerichtete Dispensiernadel 424b eingebracht, um an den Partikeln 411 und/oder der Innenwand der Küvette verbliebene bzw. unspezifisch anhaftende überschüssige Bestandteile durch vorsichtige Spülung der Partikel 411 zu entfernen, wobei die Partikel 411 nach wie vor magnetisch an der Gefäßwand gehalten werden (K12, in Fig. 4c).

Anschließend wird die Küvette 201 erneut trocken gesaugt, wobei die Partikel 411 samt den daran gebundenen Substanzen noch immer magnetisch an der Innenwand der Küvetten 201 fixiert sind (K13, in Fig. 4c). die Schritte K12 und K13 können beliebig oft wiederholt werden.

Eine zweite, vertikal ausgerichtete Dispensiernadel 424b hingegen erzeugt beim Eindüsen von Waschlösung oder Verdünnungsflüssigkeit Turbulenzen in der Flüssigkeit, sodass die Partikels 411 bei abgedockten Magneten in der Flüssigkeit resuspendiert werden (K14, in Fig. 4c).

Nach diesen Waschschritten (K11-K14), wird nach einer abschließenden magnetischen Separation und Absaugung (nicht dargestellt) der Fotomultiplier 435 an die Küvette 201 herangefahren. Durch die beiden Dispensoren 424c und 424d werden nun zunächst Pretriggerlösung (K15, in Fig. 4c) und nach einer Einwirkzeit (ca. 5 Sekunden) Triggerlösung (K16, in Fig. 4c) zugeführt. Damit wird eine nur wenige Sekunden andauernde Chemilumineszenz L (Flash Luminescence) ausgelöst, die vom Fotomultiplier 435 gemessen wird. Die hierfür auf die Füllöffnung 207 der Küvette 201 aufgesetzte Dispensorplattform 421 des Haltearms sorgt gleichzeitig für die hierzu benötigte Verdunkelung der Küvette 201.

### Single-Step kompetitiver Assay

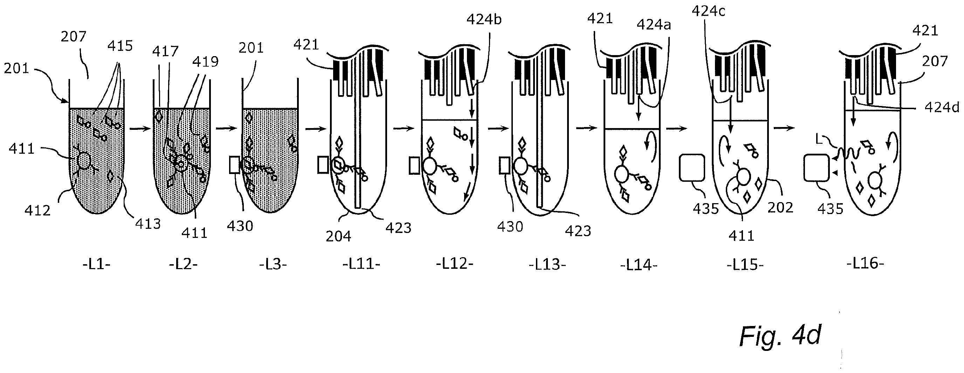

Bei einem Single-Step kompetitiven Assay gemäß Fig. 4d werden zunächst die Probe (enthält den Analyt 413), eine Suspension magnetischer Partikel 411 mit einer Beschichtung aus einem Fängerantikörper 412, sowie eine Lösung des Tracer-Antigens 415 in die Küvette 201 mittels eines hier nicht dargestellten Pipettors einpipettiert (L1, in Fig. 4d).

Während der der nachfolgenden Inkubation (ca. 10 min) bei 37 °C wird die Lösung beispielsweise mittels Ultraschalls gerührt, um ein Absinken und Agglomerieren der Partikel zu verhindern und um eine gute Durchmischung aller Komponenten zu gewährleisten. Während der Inkubationsphase konkurrieren die Analytmoleküle 413 mit den Tracer-Antigenmolekülen 415 um die freien Bindungsstellen der an den Partikeln 411 gebundenen Fängerantikörpermolekülen 412 und sind nach der Inkubationsphase entsprechend ihrem Konzentrationsverhältnis an diesen gebunden. Weiters gibt es unspezifisch gebundene Analytmoleküle 417 und Tracer-Antigene 419 an der Partikeloberfläche und/oder der Küvettenwand. (L2, in Fig. 4d).

Die Partikel 411 samt den daran gebundenen Substanzen werden nun mit Hilfe der Magnetanordnung 430 an der Innenwand der Küvette 201 fixiert (L3, in Fig. 4d)

Nach der Fixierung der Partikel 411 wird die gesamte Flüssigkeit weitgehend mit der aus der Dispensorplattform 421 abgesenkten Absaugnadel 423 entfernt (L11, in Fig. 4d).

In weiterer Folge wird eine Waschlösung durch eine schräg auf die Innenwand der Küvette 201 gerichtete Dispensiernadel 424b eingebracht, um an den Partikeln 411 und/oder der Innenwand der Küvette verbliebene bzw. unspezifisch anhaftende überschüssige Bestandteile durch vorsichtige Spülung der Partikel 411 zu entfernen, wobei die Partikels 411 nach wie vor magnetisch an der Gefäßwand gehalten werden (L12, in Fig. 4d).

Anschließend wird die Küvette 201 erneut trocken gesaugt, wobei die Partikel 411 samt den daran gebundenen Substanzen noch immer magnetisch an der Innenwand der Küvetten 201 fixiert sind (L13, in Fig. 4d).

Eine zweite, vertikal ausgerichtete Waschnadel 424a hingegen erzeugt beim Eindüsen von Waschlösung oder Verdünnungsflüssigkeit Turbulenzen in der Flüssigkeit, sodass die Partikel 411 bei abgedockten Magneten in der Flüssigkeit resuspendiert werden (L14, in Fig. 4d).

Nach diesen Waschschritten (L11-L14), wird nach einer abschließenden magnetischen Separation und Absaugung (nicht dargestellt), der Fotomultiplier 435 an die Küvette 201 herangefahren. Durch die beiden Dispensoren 424c und 424d werden nun zunächst Pretrigger- (L15, in Fig. 4d) und nach einer Einwirkzeit (ca. 5 Sekunden) Triggerlösung (L16, in Fig. 4d) zugeführt. Damit wird eine nur wenige Sekunden andauernde Chemilumineszenz L (Flash Luminescence) ausgelöst, die vom Fotomultiplier 435 gemessen wird. Die hierfür auf die Füllöffnung 207 der Küvette 201 aufgesetzte Dispensorplattform 421 des Haltearms sorgt gleichzeitig für die hierzu benötigte Verdunkelung der Küvette 201.

Die Waschschritte T5 bis T6, T12 bis T13, S12-S13, K5 bis K6, K12 bis K13 und L12 bis L13 können beliebig wiederholt werden.

Im Anschluss an die Messungen (T16, S16, K16, L16) wird die benutzte Küvette 201 mit der Absaugnadel 423 leergesaugt und entweder durch eine Wegwerfküvette ausgetauscht, oder gereinigt und wiederverwendet, sodass eine neuer Immunoassay in der zuvor genutzten Küvettenposition stattfinden kann.

Das Waschen der Küvette kann durch Einbringen von Waschflüssigkeit über die Dispensiernadeln 424a und/oder 424b und das Leersaugen und Trocknen über die Absaugnadel 423 erfolgen.

Bevorzugt wird zum Waschen der Küvette ein hier nicht näher beschriebener, auf einer eigenen Verfahrschiene entlang des Küvettenarrays verfahrbarer Küvettenwäscher eingesetzt.

Grundsätzlich können aber auch andere, etwas abgewandelte Immunoassays, die eine magnetische Separation mit (B/F) - Waschen als Prozessschritt aufweisen, mit der erfindungsgemäßen Vorrichtung durchgeführt werden, wobei für die Detektion gegebenenfalls auch eine andere Detektionsmethode als Messung der Chemolumineszenz vorgesehen sein kann.

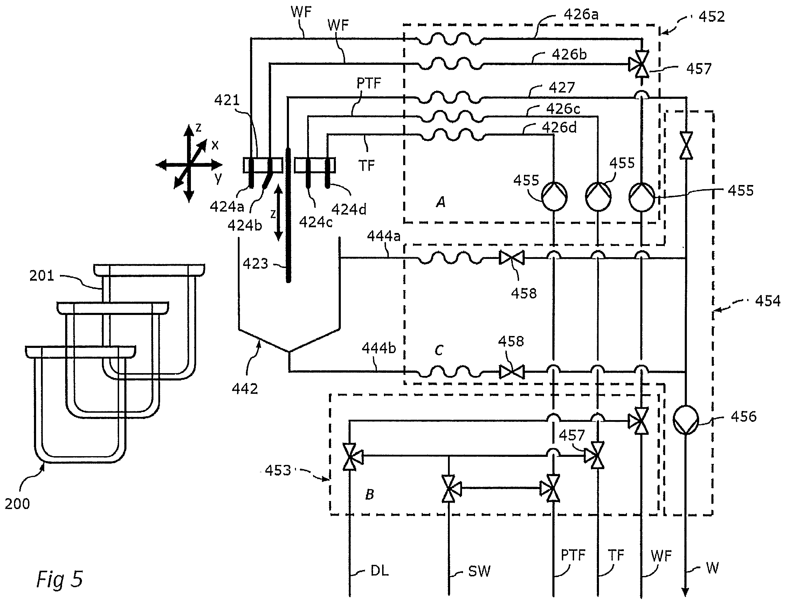

Wie in Fig. 5 schematisch dargestellt, verfügt das verfahrbare Mess- und Manipulationsmodul 450 der Erfindung über ein fluidisches System 451 zur Versorgung der Dispensorplattform 421 mit Waschflüssigkeit WF, Pretriggerflüssigkeit PTF, Triggerflüssigkeit TF und Druckluft DL. Weiters sind Einrichtungen zur Absaugung von Reaktionsgemisch oder Waschflüssigkeit aus den Küvetten 201 des Küvettenarrays 200 sowie dem Behälter, bzw. Waschtrog, der Waschstation 442 vorgesehen.

Das fluidische System 451 wird über den HetIA Controller 460 (siehe Fig. 6) gesteuert und umfasst eine Reihe von magnetisch betätigbaren 3-Wegeventilen 457 und Präzisionskolbenpumpen als Dispensierpumpen 455, welche an die verfahrbare Plattform 440 (siehe Fig. 2) über flexible Schlauchverbindungen (mit Wellenlinien angedeutet) angebunden sind.

Die über die kombinierten Freiheitsgrade der verfahrbaren Plattform 440 sowie des verschwenkbaren Haltearms 420 in x, y und z Richtung verfahrbare Dispensorplattform 421 umfasst eine Gruppe von Dispensoren 424a bis 424d, die durch die absenkbare Absaugnadel 423 ergänzt wird.

Die Dispensiereinheit 452 umfasst jeweils eine eigene Dispensierpumpe 455 für die Bereitstellung von Waschflüssigkeit WF, Pretrigger- PTF und Triggerflüssigkeit TF, wobei der Flüssigkeitsstrom aus der Dispensierpumpe 455 für die Waschflüssigkeit über ein 3-Wegeventil 457 jeweils der geraden 424a oder der schrägen Waschnadel 424b zugeschaltet werden kann. Die vier selektiv beschickbaren Versorgungsleitungen sind an den bewegbaren Stellen aus einem flexiblen Kunststoff ausgeführt und in Energieketten (nicht dargestellt) geführt.

Die Dispensierpumpen 455 der Dispensiereinheit 452 sind jeweils über eigene Versorgungsleitungen an das Ventilnetzwerk 453 angebunden, wobei für Spül- und Reinigungszwecke, insbesondere zur Reinigung der Dispensoren 424a bis 424d und der Absaugnadel 423, jeweils anstelle des primären Fördermediums alternativ auch Druckluft DL oder Systemwasser SW (deionisiertes Wasser) über ein entsprechendes 3-Wegventil 457 zugeschaltet und den Dispensierpumpen 455 zugeführt werden kann.

Der Behälter der Waschstation 442 zur Reinigung der Dispensoren 424a bis 424d und der Absaugnadel 423 weist zwei Absaugleitungen 444a, 444b auf, von denen sich eine 444b im Boden des Behälters, eine zweite in der oberen Hälfte des Behälters befindet, um als Überlauf zur Einstellung eines stabilen Füllniveaus fungieren zu können. Die Absaugeinheit 454 steht sowohl mit den beiden Absaugleitungen 444a, 444b, als auch mit der Absaugnadel 423 über flexible Schlauchleitungen in Verbindung, die in Energieketten (nicht dargestellt) geführt sind. Um einen unerwünschten Rückfluss von abgesaugten Flüssigkeiten zu unterbinden, sind jeweils Absperrventile 458 vorgesehen. Die drei Abflussleitungen münden in eine gemeinsame Zuleitung einer Absaugpumpe 456 (z.B. einer selbstansaugenden Verdrängerpumpe), die die abgesaugten Abfallflüssigkeiten W einem Sammel- oder Aufbereitungsbereich in der Vorrichtung zuführt (nicht dargestellt).

Fig. 6 zeigt ein Blockschaltbild zur elektronischen Steuerung der erfindungsgemäßen Vorrichtung gemäß Fig. 2. Der HetIA Controller 460 des Controllerboards 461 bedient die elektrischen und mechanischen Komponenten des HetIA Moduls und wird von einem Hauptrechner 588 (z.B. Personal Computer) gesteuert und programmiert. Der PC steuert Ablauf und Reihenfolge der Teilprozesse, für die Ausführung der einzelnen Aktionen ist der HetIA Controller 460 verantwortlich.

Die Funktionen des HetIA Controllers 460 können wie folgt zusammengefasst werden (siehe Fig. 6):
- Kommunikation mit PC 588 über Ethernet-Schnittstelle
- Robotikfunktionen RF mittels Schrittmotoren
   ∘ Verfahren der Plattform 440 in x-Richtung zur jeweiligen Küvette 201 des stationären Küvettenarrays 200 (bzw. zur stationären Referenzlichtquelle 436b im Küvettenblock 820, falls keine mitfahrende Referenzlichtquelle 436a vorgesehen ist)
   ∘ Drehbewegung des Rotorarms 447 zum Positionstausch von Detektionseinrichtung 435 (Fotomultiplier) und Magnetanordnung 430
   ∘ y-Bewegung für das Andocken des Fotomultipliers 435 oder der Magnetanordnung 430 an das Messfenster der Küvette 201, bzw. an eine auf der Plattform 440 mitfahrende Referenzlichtquelle 436a
   ∘ Öffnen des Shutters vor dem Detektor
- Steuerung FV der Fluidikventile 457, 458 des Fluidiksystems 451
- Steuerung DP für die Dosierpumpen 455
- Steuerung UM für den Ultraschall-Wandler 840
   ∘ Weist einen eigenen, vom Controllerboard 461 unabhängigen US-Oszillator auf
   ∘ Dekoderfunktion für die piezoelektrischen Wandler 840 an den einzelnen Küvetten 201
- Steuerung DE für die Detektionseinrichtung 435 sowie die Referenzlichtquelle 436a
- Temperaturregelung TR für die Thermostatisierung (37°C)
   ∘ Peltier Regler für die Detektionseinrichtung 435 (Fotomultiplier)
   ∘ Peltier Regler für den Küvettenblock 820

Bestimmte Funktionen, die in Echtzeit exakt synchronisiert getriggert werden müssen (siehe Klammern "S" in Fig. 6), werden im FPGA des HetIA Controllers 460 realisiert. Es sind dies beispielsweise:
- zeitlich Triggerung der Steuerung DP der Dosierpumpe zeitlich mit der Steuerung DE für die Detektionseinrichtung 435 (Fotomultiplier Messung)
- Triggerung der Referenzlichtquelle zeitlich synchron mit der Messung des Fotomultipiers
- Ultraschall Mischprozess der jeweiligen Küvette.

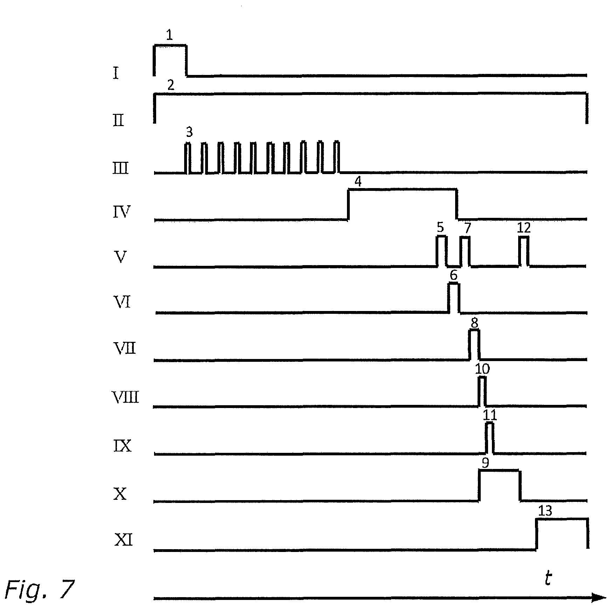

Der bereits in Fig. 4 dargelegte Ablauf eines heterogenen Immunoassays am Beispiel eines 1-Step-Sandwich-Assays wird nun aus der Sicht der dazu notwendigen maschinellen Abläufe im HetIA-Modul beschrieben. Die zeitliche Verteilung der maschinellen Aktivitäten während des Assays ist in den Linienzügen I bis XI über der gemeinsamen Zeitachse t in Fig. 7 dargestellt.
I Zupipettierung von Proben, Reagenzien und einer Beads-Suspension in die Küvette 201
II Thermostatisierung des Küvettenblocks
III Aktivierung der Ultraschall-Mischeinheit
IV Andocken der Magnetanordnung 430 zur Separation der Beads
V Aktivitäten der Absaugnadel 423
VI Waschen der fixierten Beads mit der schrägen Waschnadel 424b (laminar)
VII Re-Suspendierung der Beads mit der geraden Waschnadel 424a (turbulent)
VIII Dispensierung von Pretrigger-Flüssigkeit über den Dispensor 424c (turbulent)
IX Dispensierung von Trigger-Flüssigkeit über den Dispensor 424d (turbulent)
X Tausch der Position von Magnetanordnung 430 und Fotomultiplier 435 und Lumineszenzmessung an der Küvette 201
XI Waschen der Absaugnadel 423 und der Dispensoren 424a bis 424d

Die einzelnen Maßnahmen sind in Fig. 7 in zeitlicher Abfolge mit 1 bis 13 bezeichnet.
1) Zunächst erfolgt eine Pipettierung der Probe, einer Suspension magnetischer Partikel (magnetic Beads) mit einer Beschichtung aus einem Fängerantikörper, sowie einer Lösung des Tracer-Antikörpers in die Küvette (siehe S1, Fig. 4). Eine maschinelle Pipettierung kann beispielsweise durch einen aus dem Stand der Technik bekannten Laborroboter mit x/y/z Beweglichkeit erfolgen, während die händische Pipettierung durch einen Labortechniker, beispielsweise mittels Handpipetten mit Einwegspitzen, erfolgen kann.
2) Als ständige Hintergrundaktivität bei allen maschinellen Prozessen des Assays erfolgt eine Thermostatisierung des Küvettenblocks 820 mit den darin befindlichen Küvetten 201 auf 37°C (siehe Linie II).
3) Das Proben-Reagenziengemisch in der Küvette 201 erwärmt sich nun langsam auf die Zieltemperatur von 37 °C und wird insgesamt etwa 10 min lang inkubiert, während die Ultraschall-Mischvorrichtung das Proben-Reagenziengemisch über den Ultraschall-Wandler 840 an der Küvette 201 zunächst initial homogenisiert und in kurzen Abständen immer wieder aufrührt, um ein Absinken und Agglomerieren der Beads zu verhindern (siehe Linie III).
4) Nach erfolgter Inkubation wird die magnetische Separation der inkubierten Beads durch eine Rotation der Magnetanordnung 430 auf dem Mess- und Manipulationsmodul 450 zur entsprechenden Zugangsöffnung im Küvettenblock 820 eingeleitet (siehe Linie IV).
5) Nach erfolgter Separation der Beads an der Wand der Küvette 201 wird die Absaugnadel 423 bis nahe an den Küvettenboden abgesenkt und die gesamte Flüssigkeit in der Küvette abgesaugt (siehe S4, Fig. 2), während die Magnetanordnung 430 in der Zugangsöffnung 825 verbleibt, um die Beads weiter festzuhalten.
6) Nun wird über die schräge Waschnadel 424b laminar an der Wand der Küvette 201 fließende Waschlösung eingebracht, um überschüssige und nicht gebundene Komponenten von der Wand und den magnetisch an der Wand fixierten Beads zu waschen (siehe Linie VI).
7) Nun wird die Küvette 201 trocken gesaugt, wobei die Beads noch immer magnetisch an der Wand der Küvette 201 fixiert sind (siehe Linie V).
8) In der Folge wird über eine zweite, vertikal ausgerichtete, Turbulenzen erzeugende Waschnadel 424a erneut Waschflüssigkeit eingebracht, sodass die Beads bei abgedockter Magnetanordnung in der Flüssigkeit re-suspendiert werden (siehe Linie VII). Diesem Schritt folgt eine erneute magnetische Separation und Absaugung gemäß der Schritte 4) und 5) (nicht dargestellt).
   Die Schritte 5-8 können gegebenenfalls wiederholt werden, um eine noch bessere Abtrennung von ungebundenen Tracer-Antikörpern zu erreichen.
9) Anschließend wird die Position von Detektionseinrichtung 435 und Magnetanordnung 430 durch eine 180° Rotation getauscht und die Detektionseinrichtung in die entsprechende Zugangsöffnung im Küvettenblock 820 eingeführt (siehe Linie X).
10) Nun erfolgt zunächst die Zugabe von Pretriggerflüssigkeit über einen ersten, vertikal ausgerichteten, turbulent einspritzenden Dispensor 424c (siehe S8, Fig. 4)
11) Die Lumineszenz wird nun durch Zugabe der Triggerflüssigkeit über einen weiteren, vertikal ausgerichteten, turbulent einspritzenden Dispensor 424d ausgelöst (siehe Linie IX).
12) Nun wird die Küvette 201 mittels Küvetten-Waschvorrichtung geleert und gewaschen oder alternativ die Absaugnadel 423 erneut eingeführt und das Reaktionsgemisch abgesaugt und die Küvette 201 durch eine Wegwerfküvette ersetzt.
13) Schließlich wird der Schwenkarm um 90° horizontal zur Seite geschwenkt und in die Waschstation 442 eingeführt. Die Dispensoren 424a bis 424d sowie die Absaugnadel 423 der Dispensorplattform 421 werden gewaschen, indem aus einer der beiden Waschnadeln für das (B/F)-Waschen so lange Waschlösung einfließt, bis der Füllstand zu einer Benetzung aller Nadeln ausreicht (siehe Linie XI).

## Patentansprüche

1. Vorrichtung (410) zur Durchführung von heterogenen Immunoassays mit Hilfe magnetischer Partikel (411) in aneinandergereihten Küvetten (201), wobei jede Küvette (201) eine Füllöffnung (207) und zumindest ein seitliches, für die Messstrahlung durchlässiges Messfenster (202) aufweist,
**gekennzeichnet durch**,
zumindest ein stationäres Küvettenarray (200), in welchem die Küvetten (201) zur Aufnahme flüssiger Medien angeordnet sind,
zumindest einen, entlang des Küvettenarrays (200) verfahrbaren und in Richtung der Füllöffnung (207) einer ausgewählten Küvette (201) absenkbaren Haltearm (420) mit zumindest einer, in Richtung Boden (204) der Küvette (201) absenkbaren Absaugnadel (423), sowie mit zumindest einem, über oder in der jeweiligen Füllöffnung (207) positionierbaren Dispensor (424a bis 424d) zur Abgabe der flüssigen Medien in die Küvette (201), wobei zumindest ein Dispensor (424a, 424b) zur Abgabe einer Waschlösung für die magnetischen Partikel (411) ausgebildet ist,
zumindest eine, entlang des Küvettenarrays (200) verfahrbare, auf den Inhalt der ausgewählten Küvette (201) wirkende Magnetanordnung (430) zur Separation der magnetischen Partikel (411) an einer Innenfläche der Küvette (201), sowie
zumindest eine, entlang des Küvettenarrays (200) verfahrbare, und auf das Messfenster (202) der ausgewählten Küvette (201) ausrichtbare, optische Detektionseinrichtung (435) zur Aufnahme eines mit einer Analytkonzentration in der ausgewählten Küvette (201) korrelierenden Messsignals.

2. Vorrichtung nach Anspruch 1, **dadurch gekennzeichnet, dass** der zumindest eine Dispensor (424a bis 424d) zur Abgabe der flüssigen Medien in einer auf oder in die Füllöffnung (207) der Küvette (201) absenkbaren Dispensorplattform (421) angeordnet ist, welche von der absenkbaren Absaugnadel (423) durchsetzt wird.

3. Vorrichtung nach Anspruch 1 oder 2, **dadurch gekennzeichnet, dass** die Dispensorplattform (421) an der der Küvette (201) zugewandten Seite eine Dichtfläche (425) oder eine formschlüssige Abschottung aus einem lichtundurchlässigen Material aufweist.

4. Vorrichtung nach einem der Ansprüche 1 bis 3, **dadurch gekennzeichnet, dass** ein erster Dispensor (424a) zur Abgabe einer Waschlösung für die magnetischen Partikel (411) eine Ausströmungsrichtung aufweist, die im Wesentlichen parallel zur Längsachse der Küvette (201) ausgerichtet ist, sowie dass ein zweiter Dispensor (424b) zur Abgabe einer Waschlösung für die magnetischen Partikel (411) eine Ausströmungsrichtung aufweist, die auf eine innere Seitenfläche der Küvette (201) gerichtet ist.

5. Vorrichtung nach einem der Ansprüche 1 bis 4, **dadurch gekennzeichnet, dass** von weiteren Dispensoren (424c, 424d), deren Ausströmungsrichtungen im Wesentlichen parallel zur Längsachse der Küvette (201) ausgerichtet sind, ggf. ein dritter Dispensor (424c) zur Abgabe einer Pretriggerlösung und ein vierter Dispensor (424d) zur Abgabe einer Triggerlösung ausgebildet ist.

6. Vorrichtung nach einem der Ansprüche 1 bis 5, **dadurch gekennzeichnet, dass** der Haltearm (420) für die Absaugnadel (423) und den zumindest einen Dispensor (424a bis 424d) eine Hub- und Dreheinrichtung (445) aufweist, die auf einer entlang des Küvettenarrays (200) verfahrbaren Plattform (440) angeordnet ist.

7. Vorrichtung nach Anspruch 6, **dadurch gekennzeichnet, dass** auf der verfahrbaren Plattform (440) eine Waschstation (442) für die Absaugnadel (423) und den zumindest einen Dispensor (424a bis 424d) angeordnet ist, auf deren Öffnung (443) der Haltearm (420) nach einer Drehbewegung absenkbar ausgeführt ist.

8. Vorrichtung nach Anspruch 6 oder 7, **dadurch gekennzeichnet, dass** auf der verfahrbaren Plattform (440) eine gemeinsame Aufhängung (446) für die Magnetanordnung (430) und die Detektionseinrichtung (435) angeordnet ist.

9. Vorrichtung nach Anspruch 8, **dadurch gekennzeichnet, dass** die gemeinsame Aufhängung (446) für die Magnetanordnung (430) und die Detektionseinrichtung (435) geeignet ist, eine translatorische oder rotatorische Bewegung auszuführen, um die Positionen der Magnetanordnung (430) und der Detektionseinrichtung (435) vor der ausgewählten Küvette (201) auszutauschen.

10. Vorrichtung nach Anspruch 9, **dadurch gekennzeichnet, dass** die Magnetanordnung (430) und die Detektionseinrichtung (435) im gleichen Abstand zu einer gemeinsamen Drehachse (448) an einem in der Aufhängung (446) gelagerten Rotorarm (447) befestigt sind.

11. Vorrichtung nach Anspruch 10, **dadurch gekennzeichnet, dass** der in der Aufhängung (446) gelagerte Rotorarm (447) translatorisch in Richtung der Drehachse (448) verschiebbar ist, um die Magnetanordnung (430) oder die Detektionseinrichtung (435) an das Messfenster (202) der ausgewählten Küvette (201) heranzuführen.

12. Vorrichtung nach einem der Ansprüche 6 bis 11, **dadurch gekennzeichnet, dass** der auf der verfahrbaren Plattform (440) angeordnete Haltearm (420) samt Dispensorplattform (421) zusammen mit der Magnetanordnung (430) und der Detektionseinrichtung (435) ein entlang des Küvettenarrays (200) verfahrbares Mess- und Manipulationsmodul (450) bildet, das sämtliche robotische, fluidische und messtechnische Komponenten für die Prozessschritte eines heterogenen Immunoassays vereint.

13. Vorrichtung nach einem der Ansprüche 6 bis 12, **dadurch gekennzeichnet, dass** auf der verfahrbaren Plattform (440) eine Referenzlichtquelle (436a) für die Kalibration der Detektionseinrichtung (435) angeordnet ist.

14. Vorrichtung nach einem der Ansprüche 1 bis 13, **dadurch gekennzeichnet, dass** die Detektionseinrichtung (435) als Fotomultipier ausgeführt ist.

15. Vorrichtung nach einem der Ansprüche 1 bis 14, **dadurch gekennzeichnet, dass** die Vorrichtung eine zweite, unabhängig von der ersten Magnetanordnung (430) entlang des Küvettenarrays (200) verfahrbare, auf den Inhalt einer ausgewählten Küvette (201) wirkende Magnetanordnung aufweist.

16. Vorrichtung nach Anspruch 15, **dadurch gekennzeichnet, dass** die zweite Magnetanordnung auf einer separaten Schiene entlang des Küvettenarrays (200) verfahrbar angeordnet ist.

17. Verfahren zur Bestimmung eines Analyten in einer Probe mittels eines heterogenen Immunoassays, wobei in aneinandergereihte Küvetten (201) - jeweils aufweisend eine Füllöffnung (207) und zumindest ein seitliches Messfenster (202) - Proben und magnetische Partikel eingebracht werden, **dadurch gekennzeichnet, dass** zunächst in einer ersten Schrittfolge A
a) eine Probe zur Bestimmung des Analyten, sowie
b) eine Suspension der magnetischen Partikel mit einem Fängerantikörper
in eine ausgewählte Küvette (201) der als stationäres Küvettenarray (200) angeordneten, aneinandergereihten Küvetten (201) einpipettiert werden, sowie dass die folgenden Schritte B einer immunchemischen Analyse, wie
c) Inkubation und Rühren des Küvetteninhalts,
d) Separieren der magnetischen Partikel,
e) ein oder mehrmaliges Einbringen und Absaugen einer Waschlösung,
f) Einpipettieren eines Tracer-Antikörpers oder eines Tracer-Antigens und erneutes Ausführen der Schritte d) und e)
g) Zudosieren zumindest einer Trigger-Flüssigkeit, sowie
h) luminometrische Vermessung der Probe,
mit Hilfe zumindest eines entlang des Küvettenarrays (200) verfahrbaren Mess- und Manipulationsmoduls (450) erfolgen, das zur Durchführung einzelner oder aller Schritte d), e), g) und h) bei der ausgewählten Küvette (201) angehalten wird.

18. Verfahren zur Bestimmung eines Analyten in einer Probe mittels eines heterogenen Immunoassays, wobei in aneinandergereihte Küvetten (201) - jeweils aufweisend eine Füllöffnung (207) und zumindest ein seitliches Messfenster (202) - Proben und magnetische Partikel eingebracht werden, **dadurch gekennzeichnet, dass** zunächst in einer ersten Schrittfolge A
a) eine Probe zur Bestimmung des Analyten,
b) eine Suspension der magnetischen Partikel mit einem Fängerantikörper, sowie
c) ein Tracer-Antikörper oder ein Tracer-Antigen
in eine ausgewählte Küvette (201) der als stationäres Küvettenarray (200) angeordneten, aneinandergereihten Küvetten (201) einpipettiert werden, sowie dass die folgenden Schritte B einer immunchemischen Analyse, wie
d) Inkubation und Rühren des Küvetteninhalts,
e) Separieren der magnetischen Partikel,
f) ein oder mehrmaliges Einbringen und Absaugen einer Waschlösung,
g) Zudosieren zumindest einer Trigger-Flüssigkeit, sowie
h) luminometrische Vermessung der Probe,
mit Hilfe zumindest eines entlang des Küvettenarrays verfahrbaren Mess- und Manipulationsmoduls (450) erfolgen, das zur Durchführung einzelner oder aller Schritte e) bis h) bei der ausgewählten Küvette (201) angehalten wird.

19. Verfahren nach Anspruch 17 oder 18, **dadurch gekennzeichnet, dass** das Mess- und Manipulationsmodul (450) während des Ablaufs zeitaufwändiger Schritte bei der immunchemischen Analyse, wie Inkubation, etc., in der ausgewählten Küvette, zu zumindest einer weiteren Küvette (201) des Küvettenarrays (200) verfahren wird, um einzelne oder alle Schritte B einer immunchemischen Analyse durchzuführen.

20. Verfahren nach einem der Ansprüche 17 bis 19, **dadurch gekennzeichnet, dass** der Inhalt der benutzten Küvette (201) im Anschluss an die luminometrische Vermessung der Probe gemäß Punkt h) mittels einer Absaugnadel (423) leergesaugt und die Küvette (201) entweder durch eine Wegwerfküvette ersetzt oder gereinigt und wiederverwendet wird.

21. Verfahren nach Anspruch 20, **dadurch gekennzeichnet, dass** die Küvette (201) durch Einbringen einer Waschflüssigkeit mittels einer Dispensiernadel (424a, 424b) sowie Leersaugen und Trocknen mittels einer Absaugnadel (423) gereinigt wird.

22. Verfahren nach Anspruch 20, **dadurch gekennzeichnet, dass** die Küvette (201) mit Hilfe eines entlang des Küvettenarrays (200) verfahrbaren Küvettenwäschers gereinigt wird.

## Claims

1. Device (410) for carrying out heterogeneous immunoassays by means of magnetic particles (411) in lined-up cuvettes (201), wherein each cuvette (201) has a filling opening (207) and at least one lateral measurement window (202) which is transparent to the measurement radiation,
**characterised by**
at least one stationary cuvette array (200), in which the cuvettes (201) for receiving liquid media are arranged,
at least one support arm (420) which is movable along the cuvette array (200) and which is lowerable toward the filling opening (207) of a selected cuvette (201), said support arm having at least one aspirating needle (423) which is lowerable toward the bottom (204) of the cuvette (201), and also having at least one dispenser (424a to 424d), which can be positioned above or in the respective filling opening (207), for dispensing the liquid media into the cuvette (201), wherein at least one dispenser (424a, 424b) is designed to dispense a washing solution for the magnetic particles (411),
at least one magnet assembly (430) for separating the magnetic particles (411) on an inner surface of the cuvette (201), said magnet assembly being movable along the cuvette array (200) and acting on the contents of the selected cuvette (201), and
at least one optical detection device (435) for receiving a measurement signal that correlates with an analyte concentration in the selected cuvette (201), said optical detection device being movable along the cuvette array (200) and being alignable with the measurement window (202) of the selected cuvette (201).

2. Device according to claim 1, **characterised in that** the at least one dispenser (424a to 424d) for dispensing the liquid media is arranged in a dispenser platform (421) which can be lowered onto or into the filling opening (207) of the cuvette (201), the lowerable aspirating needle (423) passing through said dispenser platform.

3. Device according to claim 1 or 2, **characterised in that** the dispenser platform (421) has, on the side facing toward the cuvette (201), a sealing surface (425) or a form-fitting partition made of a material that is impervious to light.

4. Device according to any one of claims 1 to 3, **characterised in that** a first dispenser (424a) for dispensing a washing solution for the magnetic particles (411) has an outflow direction which is oriented substantially parallel to the longitudinal axis of the cuvette (201), and **in that** a second dispenser (424b) for dispensing a washing solution for the magnetic particles (411) has an outflow direction which is directed onto an inner lateral surface of the cuvette (201).

5. Device according to any one of claims 1 to 4, **characterised in that**, of further dispensers (424c, 424d), the outflow directions of which are oriented substantially parallel to the longitudinal axis of the cuvette (201), optionally a third dispenser (424c) is designed to dispense a pretrigger solution and a fourth dispenser (424d) is designed to dispense a trigger solution.

6. Device according to any one of claims 1 to 5, **characterised in that** the support arm (420) for the aspirating needle (423) and the at least one dispenser (424a to 424d) has a lifting and rotating device (445) which is arranged on a platform (440) that is movable along the cuvette array (200).

7. Device according to claim 6, **characterised in that** a washing station (442) for the aspirating needle (423) and the at least one dispenser (424a to 424d) is arranged on the movable platform (440), the support arm (420) being lowerable onto the opening (443) of said washing station after a rotational movement.

8. Device according to claim 6 or 7, **characterised in that** a common suspension mount (446) for the magnet assembly (430) and the detection device (435) is arranged on the movable platform (440).

9. Device according to claim 8, **characterised in that** the common suspension mount (446) for the magnet assembly (430) and the detection device (435) is suitable for carrying out a translational or rotational movement in order to swap the positions of the magnet assembly (430) and the detection device (435) in front of the selected cuvette (201).

10. Device according to claim 9, **characterised in that** the magnet assembly (430) and the detection device (435) are attached to a rotor arm (447), which is mounted in the suspension mount (446), at an equal distance from a common axis of rotation (448).

11. Device according to claim 10, **characterised in that** the rotor arm (447) mounted in the suspension mount (446) is movable in translation in the direction of the axis of rotation (448), in order to move the magnet assembly (430) or the detection device (435) toward the measurement window (202) of the selected cuvette (201).

12. Device according to any one of claims 6 to 11, **characterised in that** the support arm (420) arranged on the movable platform (440) forms, along with the dispenser platform (421) together with the magnet assembly (430) and the detection device (435), a measurement and manipulation module (450) which is movable along the cuvette array (200) and which combines all the robotic, fluidic and metrological components for the process steps of a heterogeneous immunoassay.

13. Device according to any one of claims 6 to 12, **characterised in that** a reference light source (436a) for calibrating the detection device (435) is arranged on the movable platform (440).

14. Device according to any one of claims 1 to 13, **characterised in that** the detection device (435) is designed as a photomultiplier.

15. Device according to any one of claims 1 to 14, **characterised in that** the device has a second magnet assembly which, independently of the first magnet assembly (430), is movable along the cuvette array (200) and acts on the contents of a selected cuvette (201).

16. Device according to claim 15, **characterised in that** the second magnet assembly is arranged such as to be movable along the cuvette array (200) on a separate rail.

17. Method for determining an analyte in a sample by means of a heterogeneous immunoassay, wherein samples and magnetic particles are introduced into lined-up cuvettes (201) which each have a filling opening (207) and at least one lateral measurement window (202), **characterised in that**, in a first step sequence A, firstly
a) a sample for determining the analyte, and
b) a suspension of the magnetic particles containing a capture antibody
are pipetted into a selected cuvette (201) of the lined-up cuvettes (201) arranged as a stationary cuvette array (200), and **in that** the following steps B of an immunochemical analysis, such as
c) incubating and stirring the cuvette contents,
d) separating the magnetic particles,
e) introducing and aspirating a washing solution one or more times,
f) pipetting in a tracer antibody or a tracer antigen and repeating steps d) and e),
g) adding a metered quantity of at least one trigger liquid, and
h) carrying out a luminometric measurement of the sample,
take place by means of at least one measurement and manipulation module (450) which is movable along the cuvette array (200) and which is stopped at the selected cuvette (201) in order to carry out some or all steps d), e), g) and h).

18. Method for determining an analyte in a sample by means of a heterogeneous immunoassay, wherein samples and magnetic particles are introduced into lined-up cuvettes (201) which each have a filling opening (207) and at least one lateral measurement window (202), **characterised in that**, in a first step sequence A, firstly
a) a sample for determining the analyte,
b) a suspension of the magnetic particles containing a capture antibody, and
c) a tracer antibody or a tracer antigen
are pipetted into a selected cuvette (201) of the lined-up cuvettes (201) arranged as a stationary cuvette array (200), and **in that** the following steps B of an immunochemical analysis, such as
d) incubating and stirring the cuvette contents,
e) separating the magnetic particles,
f) introducing and aspirating a washing solution one or more times,
g) adding a metered quantity of at least one trigger liquid, and
h) carrying out a luminometric measurement of the sample,
take place by means of at least one measurement and manipulation module (450) which is movable along the cuvette array and which is stopped at the selected cuvette (201) in order to carry out some or all steps e) to h).

19. Method according to claim 17 or 18, **characterised in that**, while time-consuming steps for the immunochemical analysis, such as incubation, etc., are being carried out in the selected cuvette, the measurement and manipulation module (450) is moved to at least one further cuvette (201) of the cuvette array (200) in order to carry out some or all steps B of an immunochemical analysis.

20. Method according to any one of claims 17 to 19, **characterised in that**, after the luminometric measurement of the sample in point h), the content of the used cuvette (201) is sucked empty by means of an aspirating needle (423) and the cuvette (201) either is replaced by a disposable cuvette or is cleaned and reused.

21. Method according to claim 20, **characterised in that** the cuvette (201) is cleaned by introducing a washing liquid by means of a dispensing needle (424a, 424b) and emptying and drying by means of an aspirating needle (423).

22. Method according to claim 20, **characterised in that** the cuvette (201) is cleaned by means of a cuvette washer which is movable along the cuvette array (200).

## Revendications

1. Dispositif (410) pour effectuer des dosages immunologiques hétérogènes à l'aide de particules magnétiques (411) dans des cuvettes (201) alignées, chaque cuvette (201) ayant un orifice de remplissage (207) et au moins une fenêtre de mesure (202), latérale, perméable au rayonnement de mesure,
dispositif **caractérisé par**
- au moins une série de cuvettes fixes (200) avec des cuvettes (201) pour recevoir des milieux liquides,
- au moins un bras de fixation (420) mobile le long de la série de cuvettes (200) et qui peut être abaissé dans la direction de l'orifice de remplissage (207) d'une cuvette sélectionnée (201), ayant au moins une aiguille d'aspiration (423) qui peut être descendue dans la direction du fond (204) de la cuvette (201) ainsi qu'au moins un distributeur (424a, 424d) se positionnant au-dessus ou dans l'orifice de remplissage (207) respectif pour distribuer les milieux liquides dans la cuvette (201), au moins un distributeur (424a, 424b) étant réalisé pour distribuer une solution de lavage pour les particules magnétiques (411),
- au moins un dispositif magnétique (430) mobile le long de la série de cuvettes (200) et agissant sur le contenu de la cuvette (201) sélectionnée pour séparer les particules magnétiques (411) sur une surface intérieure de la cuvette (201), et
- au moins une installation de détection optique (435), mobile le long de la série de cuvettes (200) et alignée sur la fenêtre de mesure (202) de la cuvette (201) sélectionnée, pour recevoir un signal de mesure en corrélation avec la concentration d'analyte dans la cuvette sélectionnée (201).

2. Dispositif selon la revendication 1,
**caractérisé en ce que**
le distributeur (424a-424d) est sur une plateforme de distributeur (421), pour distribuer les milieux liquides qui peut être descendu sur ou dans l'orifice de remplissage (207) de la cuvette (201), cette plateforme (421) étant traversée par l'aiguille d'aspiration (423) qui peut être descendue.

3. Dispositif selon la revendication 1 ou 2,
**caractérisé en ce que**
sur le côté tourné vers la cuvette (201), la plateforme de distribution (421) présente une surface d'étanchéité (425) ou une protection par la forme en un matériau perméable à la lumière.

4. Dispositif selon l'une des revendications 1 à 3,
**caractérisé en ce qu'**il comporte
un premier distributeur (424a) pour distribuer une solution de lavage des particules magnétiques (411) dans une direction de jet qui est pratiquement parallèle à l'axe longitudinal de la cuvette (201) ainsi qu'un second distributeur (424b) pour distribuer une solution de lavage des particules magnétiques (411) avec une direction de jet orientée vers la surface latérale intérieure de la cuvette (201).

5. Dispositif selon l'une des revendications 1 à 4,
**caractérisé en ce que**
parmi d'autres distributeurs (424c, 424d) dont les directions de jet sont pratiquement parallèles à l'axe longitudinal de la cuvette (201), le cas échéant, un troisième distributeur (424c) est réalisé pour distribuer une solution de pré-déclenchement ainsi qu'un quatrième distributeur (424d) pour distribuer une solution de déclenchement.

6. Dispositif selon l'une des revendications 1 à 5,
**caractérisé en ce que**
le bras de fixation (420) de l'aiguille d'aspiration (423) et au moins le distributeur (424a-424d) présentent une installation de translation et de rotation (445) sur une plateforme (440) mobile le long de la série de cuvettes (200).

7. Dispositif selon la revendication 6,
**caractérisé en ce que**
un poste de lavage (442) est prévu sur la plateforme mobile (440) pour l'aiguille d'aspiration (423) et le distributeur (424a-424d) et sur l'orifice (443) duquel le bras de maintien (420) peut s'abaisser après un mouvement de rotation.

8. Dispositif selon la revendication 6 ou 7,
**caractérisé en ce qu'**il comprend
une suspension (446) commune pour le dispositif magnétique (430) et l'installation de détection (435) sur la plateforme mobile (440).

9. Dispositif selon la revendication 8,
**caractérisé en ce que**
la suspension commune (446) du dispositif magnétique (430) et de l'installation de détection (435) peut effectuer un mouvement de translation ou de rotation pour échanger les positions du dispositif magnétique (430) et de l'installation de détection (435) devant la cuvette (201) sélectionnée.

10. Dispositif selon la revendication 9,
**caractérisé en ce que**
le dispositif magnétique (430) et l'installation de détection (435) sont fixées à équidistance d'un axe de rotation commun (448) à un bras de rotor (447) monté dans la suspension (446).

11. Dispositif selon la revendication 10,
**caractérisé en ce que**
le bras de rotor (447) monté dans la suspension (446) peut coulisser en translation dans la direction de l'axe de rotation (448) pour rapprocher le dispositif magnétique (430) ou l'installation de détection (435) de la fenêtre de mesure (202) de la cuvette sélectionnée (201).

12. Dispositif selon l'une des revendications 6 à 11,
**caractérisé en ce que**
le bras de fixation (420) monté sur la plateforme mobile (440) avec la plateforme de distributeur (421) et le dispositif magnétique (430) ainsi que l'installation de détection (435) forment un module de mesure et de manipulation (450) mobile le long de la série de cuvettes (200) et qui combine l'ensemble des composants robotiques, fluidiques et de métrologie pour les étapes du procédé pour un dosage immunologique hétérogène.

13. Dispositif selon l'une des revendications 6 à 12,
**caractérisé en ce que**
la plateforme mobile (440) comporte une source de lumière de référence (436a) pour le calibrage de l'installation de détection (435).

14. Dispositif selon l'une des revendications 1 à 13,
**caractérisé en ce que**
l'installation de détection (435) est un photomultiplicateur.

15. Dispositif selon l'une des revendications 1 à 14,
**caractérisé en ce qu'**il comporte
un second dispositif magnétique indépendant du premier dispositif magnétique (430) pour être déplacé le long de la série de cuvettes (200) et agir sur le contenu de la cuvette sélectionnée (201).

16. Dispositif selon la revendication 15,
**caractérisé en ce que**
le second dispositif magnétique est mobile sur un rail distinct le long de la série de cuvettes (200).

17. Procédé pour déterminer un analyte dans un échantillon à l'aide d'un dosage immunologique hétérogène, dans des cuvettes alignées (201) - ayant respectivement un orifice de remplissage (207) et au moins une fenêtre de mesure latérale (202) - on introduit des échantillons et des particules magnétiques,
procédé **caractérisé en ce que**
tout d'abord dans une première succession d'étapes A :
on introduit dans une cuvette sélectionnée (201) d'un série de cuvettes fixes (200) composée de cuvettes alignées (201),
a) un échantillon pour déterminer l'analyte, et
b) une suspension de particules magnétiques avec un anticorps capteur, dans les étapes suivantes B d'un analyse immunochimique,
c) incubation et agitage du contenu des cuvettes,
d) séparation des particules magnétiques,
e) introduction en une ou plusieurs fois et aspiration d'une solution de lavage,
f) introduction par pipette d'un anticorps traceur ou d'un antigène traceur et répétition des étapes d) et e),
g) dosage d'au moins un liquide de déclenchement, ainsi que
h) mesure de l'échantillon de manière luminométrique,
opération faite à l'aide d'au moins un module de mesure et de manipulation (450) mobile le long de la série de cuvettes (200), pour effectuer certaines ou toutes les étapes d), e), g), et h) sur la cuvette sélectionnée (201).

18. Procédé pour déterminer un analyte dans un échantillon à l'aide d'un dosage immunologique hétérogène, dans des cuvettes alignées (201) - ayant respectivement un orifice de remplissage (207) et au moins une fenêtre de mesure (202) latérale - on introduit des échantillons et des particules magnétiques,
procédé **caractérisé en ce que**
tout d'abord dans une première succession d'étapes A :
on introduit dans une cuvette sélectionnée (201) d'un série de cuvettes fixes (200) composée de cuvettes alignées (201),
a) un échantillon pour déterminer l'analyte,
b) une suspension de particules magnétiques avec un anticorps capteur, et
c) un anticorps traceur ou un antigène traceur, et
dans les étapes suivantes B d'un analyse immunochimique avec au moins un module de mesure et de manipulation (450) mobile le long de la série d'échantillons, on s'arrête pour effectuer certaines ou toutes les étapes e)-h) sur la cuvette sélectionnée (201) à savoir,
d) incubation et agitation du contenu de la cuvette,
e) séparation des particules magnétiques,
f) introduction et aspiration, une ou plusieurs fois, d'une solution de lavage,
g) dosage d'au moins un liquide de déclenchement, et
h) mesure luminométrique de l'échantillon.

19. Procédé selon la revendication 17 ou 18,
**caractérisé en ce que**
pendant le déroulement d'étapes longues de l'analyse immunochimique dans la cuvette sélectionnée, on déplace le module de mesure et de manipulations (450) vers au moins une autre cuvette (201) de la série de cuvettes (200) pour effectuer certaines ou toutes les étapes B de l'analyse immunochimique.

20. Procédé selon l'une des revendications 17 à 19,
**caractérisé en ce que**
après la mesure luminométrique de l'échantillon selon l'étape h), on vide par aspiration le contenu de la cuvette utilisée (201) à l'aide d'une aiguille aspirante (423) et on remplace la cuvette (201) par une cuvette jetable, ou on la nettoie et on la réutilise.

21. Procédé selon la revendication 20,
**caractérisé en ce que**
on vide par aspiration la cuvette (201) en y introduisant un liquide de lavage à l'aide d'une aiguille de distribution (424a, 424b) et on sèche en nettoyant à l'aide d'une aiguille d'aspiration (423).

22. Procédé selon la revendication 20,
**caractérisé en ce que**
on nettoie la cuvette (201) à l'aide d'un dispositif de lavage de cuvettes, mobile le long de la série de cuvettes (200).
